(19) Europäisches Patentamt
European Patent Office
Office européen des brevets

(11) EP 4 253 388 A1

(12) # EUROPEAN PATENT APPLICATION
published in accordance with Art. 153(4) EPC

(43) Date of publication:
**04.10.2023 Bulletin 2023/40**

(21) Application number: **21897229.7**

(22) Date of filing: **30.11.2021**

(51) International Patent Classification (IPC):
***C07D 519/00*** *(2006.01)* ***A61K 31/519*** *(2006.01)*
***A61P 35/00*** *(2006.01)*

(52) Cooperative Patent Classification (CPC):
**A61K 31/519; A61P 35/00; C07D 519/00**

(86) International application number:
**PCT/CN2021/134435**

(87) International publication number:
**WO 2022/111729 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.11.2020 CN 202011383418**

(71) Applicant: **CHIA TAI TIANQING PHARMACEUTICAL GROUP CO., LTD.**
**Lianyungang,**
**Jiangsu 222062 (CN)**

(72) Inventors:
- **LI, Gang**
  **Shanghai 200131 (CN)**
- **LU, Lun**
  **Shanghai 200131 (CN)**
- **HU, Lihong**
  **Shanghai 200131 (CN)**
- **DING, Charles Z.**
  **Shanghai 200131 (CN)**
- **CHEN, Shuhui**
  **Shanghai 200131 (CN)**

(74) Representative: **v. Bezold & Partner Patentanwälte - PartG mbB**
**Ridlerstraße 57**
**80339 München (DE)**

(54) **SALT FORM USED AS CDC7 INHIBITOR AND CRYSTAL FORM THEREOF**

(57) Disclosed are a salt form used as a CDC7 inhibitor and a crystal form thereof, and specifically disclosed is a compound represented by formula (II), a crystal form thereof, a preparation method thereof, and an application thereof in the preparation of a drug for preventing or treating tumors.

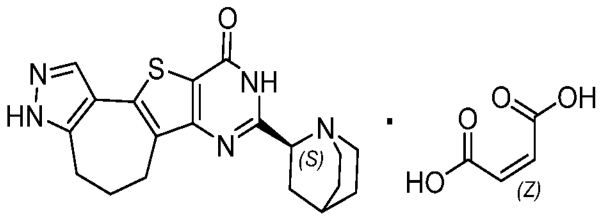

(II)

EP 4 253 388 A1

## Description

### CROSS-REFERENCE TO RELATED APPLICATIONS

The present disclosure claims priority and benefit to the Chinese Patent Application No. 202011383418.7 filed with National Intellectual Property Administration, PRC on Nov. 30, 2020, the disclosure of which is incorporated herein by reference in its entirety.

### TECHNICAL FIELD

The present disclosure is in the field of pharmaceutical chemistry, and particularly relates to a salt form as a Cdc7 inhibitor and crystal forms thereof, more particularly to a compound of formula (II) and a crystal form thereof, and also to a method for preparing the salt form and the crystal form and use thereof for preparing a medicament for preventing or treating tumors.

### BACKGROUND

Cdc7 is a serine/threonine kinase that binds to ASK (activator of S phase kinase, also known as DBF4) in the nucleus. In one aspect, it activates MCM to promote the formation of replication origin complexes by phosphorylating minichromosome maintenance proteins (MCM proteins), an important element of DNA replication initiator. In another aspect, it also participates in the ATR/Chk1 pathway and serves as an important regulatory factor of the S-phase checkpoint of a cell cycle to promote cell cycle arrest and DNA repair. Cdc7 is abnormally highly expressed in various human tumor cells, and such abnormally high expression is shown to highly correlate with tumor proliferation and metastasis as well as resistance to chemotherapeutic drugs. Therefore, Cdc7 has now become an important marker and target in tumor research.

### BRIEF SUMMARY

In one aspect, the present disclosure provides a compound of formula (II)

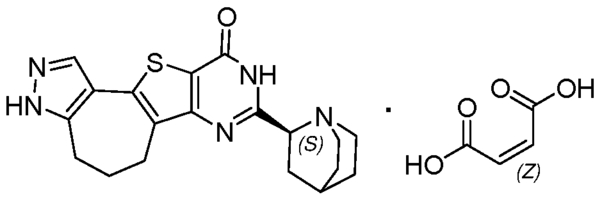

(II)

.

In another aspect, the present disclosure also provides a crystal form of the compound of formula (II).

In some embodiments of the present disclosure, the crystal form of the compound of formula (II) is selected from the group consisting of:

crystal form A of the compound of formula (II), an X-ray powder diffraction pattern of which comprises characteristic peaks at 2θ values of 11.46 ± 0.20°, 24.03 ± 0.20° and 25.16 ± 0.20°;

crystal form B of the compound of formula (II), an X-ray powder diffraction pattern of which comprises characteristic peaks at 2θ values of 5.86 ± 0.20°, 17.64 ± 0.20° and 24.89 ± 0.20°; and

crystal form C of the compound of formula (II), an X-ray powder diffraction pattern of which comprises characteristic peaks at 2θ values of 7.40 ± 0.20°, 11.21 ± 0.20° and 22.18 ± 0.20°.

In another aspect, the present disclosure also provides crystal form A of the compound of formula (II), an X-ray powder diffraction pattern of which comprises characteristic peaks at 2Θ values of 11.46 ± 0.20°, 24.03 ± 0.20° and 25.16 ± 0.20°.

In some embodiments of the present disclosure, an X-ray powder diffraction pattern of the crystal form A described above using Cu Kα radiation has diffraction peaks at 2Θ values of 11.46 ± 0.20°, 24.03 ± 0.20° and 25.16 ± 0.20°.

In some embodiments of the present disclosure, an X-ray powder diffraction pattern of the crystal form A

described above comprises characteristic peaks at 2Θ values of 11.46 ± 0.20°, 12.06 ± 0.20°, 16.96 ± 0.20°, 17.60 ± 0.20°, 18.48 ± 0.20°, 19.55 ± 0.20°, 24.03 ± 0.20° and 25.16 ± 0.20°.

In some embodiments of the present disclosure, an X-ray powder diffraction pattern of the crystal form A described above comprises characteristic peaks at 2Θ values of 6.46° ± 0.20°, 9.36° ± 0.20°, 11.46° ± 0.20°, 12.06° ± 0.20°, 12.39° ± 0.20°, 12.89° ± 0.20°, 13.37° ± 0.20°, 13.87° ± 0.20°, 14.09° ± 0.20°, 16.10° ± 0.20°, 16.96° ± 0.20°, 17.19° ± 0.20°, 17.60° ± 0.20°, 18.48° ± 0.20°, 18.75° ± 0.20°, 19.37° ± 0.20°, 19.55° ± 0.20°, 21.21° ± 0.20°, 21.65° ± 0.20°, 22.36° ± 0.20°, 23.06° ± 0.20°, 23.42° ± 0.20°, 23.74° ± 0.20°, 24.03° ± 0.20°, 25.16° ± 0.20°, 25.47° ± 0.20°, 26.59° ± 0.20°, 27.32° ± 0.20°, 28.11° ± 0.20°, 28.77° ± 0.20°, 29.73° ± 0.20°, 31.81° ± 0.20°, 33.09° ± 0.20°, 33.80° ± 0.20°, 34.19° ± 0.20°, 35.49° ± 0.20°, 35.99° ± 0.20°, 37.66° ± 0.20°, 38.14° ± 0.20°, 38.77° ± 0.20° and 39.38° ± 0.20°.

In some embodiments of the present disclosure, an X-ray powder diffraction pattern of the crystal form A described above comprises characteristic peaks at 2Θ values of 6.46°, 9.36°, 11.46°, 12.06°, 12.39°, 12.89°, 13.37°, 13.87°, 14.09°, 16.10°, 16.96°, 17.19°, 17.60°, 18.48°, 18.75°, 19.37°, 19.55°, 21.21°, 21.65°, 22.36°, 23.06°, 23.42°, 23.74°, 24.03°, 25.16°, 25.47°, 26.59°, 27.32°, 28.11°, 28.77°, 29.73°, 31.81°, 33.09°, 33.80°, 34.19°, 35.49°, 35.99°, 37.66°, 38.14°, 38.77° and 39.38°.

In some embodiments of the present disclosure, an X-ray powder diffraction pattern of the crystal form A described above is shown in FIG. 1.

In some embodiments of the present disclosure, an X-ray powder diffraction pattern of crystal form A of the compound of formula (II) is shown in Table 1.

**Table 1. XRPD pattern analysis data of crystal form A of the compound of formula (II)**

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 1 | 6.46 | 13.69 | 4.02 | 22 | 23.42 | 3.80 | 6.95 |
| 2 | 9.36 | 9.45 | 11.84 | 23 | 23.74 | 3.75 | 10.34 |
| 3 | 11.46 | 7.72 | 50.71 | 24 | 24.03 | 3.70 | 100.00 |
| 4 | 12.06 | 7.34 | 22.16 | 25 | 25.16 | 3.54 | 29.42 |
| 5 | 12.39 | 7.14 | 4.33 | 26 | 25.47 | 3.50 | 5.01 |
| 6 | 12.89 | 6.87 | 3.05 | 27 | 26.59 | 3.35 | 1.75 |
| 7 | 13.37 | 6.62 | 7.19 | 28 | 27.32 | 3.26 | 6.12 |
| 8 | 13.87 | 6.39 | 6.58 | 29 | 28.11 | 3.17 | 5.99 |
| 9 | 14.09 | 6.29 | 6.09 | 30 | 28.77 | 3.10 | 2.43 |
| 10 | 16.10 | 5.51 | 5.75 | 31 | 29.73 | 3.01 | 2.79 |
| 11 | 16.96 | 5.23 | 15.15 | 32 | 31.81 | 2.81 | 2.39 |
| 12 | 17.19 | 5.16 | 14.98 | 33 | 33.09 | 2.71 | 1.82 |
| 13 | 17.60 | 5.04 | 15.82 | 34 | 33.80 | 2.65 | 1.63 |
| 14 | 18.48 | 4.80 | 18.58 | 35 | 34.19 | 2.62 | 2.12 |
| 15 | 18.75 | 4.73 | 5.85 | 36 | 35.49 | 2.53 | 1.08 |
| 16 | 19.37 | 4.58 | 11.94 | 37 | 35.99 | 2.50 | 1.06 |
| 17 | 19.55 | 4.54 | 13.94 | 38 | 37.66 | 2.39 | 1.81 |
| 18 | 21.21 | 4.19 | 9.40 | 39 | 38.14 | 2.36 | 0.74 |
| 19 | 21.65 | 4.11 | 6.66 | 40 | 38.77 | 2.32 | 1.55 |
| 20 | 22.36 | 3.98 | 6.35 | 41 | 39.38 | 2.29 | 1.41 |
| 21 | 23.06 | 3.86 | 6.57 | | | | |

In some embodiments of the present disclosure, a DSC curve of the crystal form A described above has an endothermic peak starting point at 230.7 °C ± 3 °C.

In some embodiments of the present disclosure, a DSC profile of the crystal form A described above is shown in FIG. 4.

In some embodiments of the present disclosure, a TGA curve of the crystal form A described above shows a weight loss of 0.94 ± 0.2% at 200.0 °C ± 3 °C.

In some embodiments of the present disclosure, a TGA profile of the crystal form A described above is shown in FIG. 5.

In some embodiments of the present disclosure, an infrared spectrum of the crystal form A described above comprises characteristic absorption peaks at 3033.88 $cm^{-1}$ ± 5 $cm^{-1}$, 3225.69 $cm^{-1}$ ± 5 $cm^{-1}$, 3087.88 $cm^{-1}$ ± 5 $cm^{-1}$, 2945.00 $cm^{-1}$ ± 5 $cm^{-1}$, 2925.62 $cm^{-1}$ ± 5 $cm^{-1}$, 2837.85 $cm^{-1}$ ± 5 $cm^{-1}$, 1680.20 $cm^{-1}$ ± 5 $cm^{-1}$, 1662.75 $cm^{-1}$ ± 5 $cm^{-1}$, 1582.38 $cm^{-1}$ ± 5 $cm^{-1}$, 1551.77 $cm^{-1}$ ± 5 $cm^{-1}$, 1475.47 $cm^{-1}$ ± 5 $cm^{-1}$ and 1438.43 $cm^{-1}$ ± 5 $cm^{-1}$.

In some embodiments of the present disclosure, an infrared absorption spectrum of the crystal form A described above is shown in FIG. 6.

In some embodiments of the present disclosure, an ultraviolet absorption spectrum of the crystal form A described above has characteristic absorption peaks at 259.0 nm ± 3 nm, 214.4 nm ± 3 nm and 322.6 nm ± 3 nm.

In some embodiments of the present disclosure, an ultraviolet absorption spectrum of the crystal form A described above is shown in FIG. 11.

In another aspect, the present disclosure also provides crystal form B of the compound of formula (II), an X-ray powder diffraction pattern of which comprises characteristic peaks at 2Θ values of 5.86 ± 0.20°, 17.64 ± 0.20° and 24.89 ± 0.20°.

In some embodiments of the present disclosure, an X-ray powder diffraction pattern of the crystal form B described above using Cu Kα radiation has diffraction peaks at 2Θ values of 5.86 ± 0.20°, 17.64 ± 0.20° and 24.89 ± 0.20°.

In some embodiments of the present disclosure, an X-ray powder diffraction pattern of the crystal form B described above comprises characteristic peaks at 2θ values of 5.86 ± 0.20°, 10.17 ± 0.20°, 11.73 ± 0.20°, 15.83 ± 0.20°, 17.64 ± 0.20°, 23.59 ± 0.20°, 24.89 ± 0.20° and 25.80 ± 0.20°.

In some embodiments of the present disclosure, an X-ray powder diffraction pattern of the crystal form B described above comprises characteristic peaks at 2Θ values of 5.86° ± 0.20°, 10.17° ± 0.20°, 11.73° ± 0.20°, 12.57° ± 0.20°, 14.15° ± 0.20°, 14.40° ± 0.20°, 15.23° ± 0.20°, 15.83° ± 0.20°, 16.26° ± 0.20°, 16.71° ± 0.20°, 17.64° ± 0.20°, 18.04° ± 0.20°, 18.73° ± 0.20°, 19.99° ± 0.20°, 20.57° ± 0.20°, 21.08° ± 0.20°, 23.59° ± 0.20°, 24.36° ± 0.20°, 24.89° ± 0.20°, 25.41° ± 0.20°, 25.80° ± 0.20°, 27.20° ± 0.20°, 27.90° ± 0.20°, 28.90° ± 0.20°, 29.39° ± 0.20°, 29.70° ± 0.20°, 30.52° ± 0.20°, 30.81° ± 0.20°, 31.99° ± 0.20°, 34.29° ± 0.20°, 35.83° ± 0.20°, 39.64° ± 0.20°, 28.90° ± 0.20°, 29.39° ± 0.20°, 29.70° ± 0.20°, 30.52° ± 0.20°, 30.81° ± 0.20°, 31.99° ± 0.20°, 34.29° ± 0.20°, 35.83° ± 0.20° and 39.64° ± 0.20°.

In some embodiments of the present disclosure, an X-ray powder diffraction pattern of the crystal form B described above comprises characteristic peaks at 2Θ values of 5.86°, 10.17°, 11.73°, 12.57°, 14.15°, 14.40°, 15.23°, 15.83°, 16.26°, 16.71°, 17.64°, 18.04°, 18.73°, 19.99°, 20.57°, 21.08°, 23.59°, 24.36°, 24.89°, 25.41°, 25.80°, 27.20°, 27.90°, 28.90°, 29.39°, 29.70°, 30.52°, 30.81°, 31.99°, 34.29°, 35.83°, 39.64°, 28.90°, 29.39°, 29.70°, 30.52°, 30.81°, 31.99°, 34.29°, 35.83° and 39.64°.

In some embodiments of the present disclosure, an X-ray powder diffraction pattern of the crystal form B described above is shown in FIG. 2.

In some embodiments of the present disclosure, an X-ray powder diffraction pattern of crystal form B of the compound of formula (II) is shown in Table 2.

**Table 2. XRPD pattern analysis data of crystal form B of the compound of formula (II)**

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 1 | 5.86 | 15.09 | 91.11 | 22 | 27.20 | 3.28 | 5.62 |
| 2 | 10.17 | 8.70 | 45.62 | 23 | 27.90 | 3.20 | 5.66 |
| 3 | 11.73 | 7.55 | 22.38 | 24 | 28.90 | 3.09 | 7.73 |
| 4 | 12.57 | 7.04 | 4.09 | 25 | 29.39 | 3.04 | 4.33 |
| 5 | 14.15 | 6.26 | 13.40 | 26 | 29.70 | 3.01 | 7.45 |
| 6 | 14.40 | 6.15 | 16.28 | 27 | 30.52 | 2.93 | 4.19 |
| 7 | 15.23 | 5.82 | 4.48 | 28 | 30.81 | 2.90 | 4.70 |

(continued)

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 8 | 15.83 | 5.60 | 16.95 | 29 | 31.99 | 2.80 | 2.25 |
| 9 | 16.26 | 5.45 | 5.96 | 30 | 34.29 | 2.61 | 3.87 |
| 10 | 16.71 | 5.30 | 9.51 | 31 | 35.83 | 2.51 | 4.54 |
| 11 | 17.64 | 5.03 | 100.00 | 32 | 39.64 | 2.27 | 4.83 |
| 12 | 18.04 | 4.92 | 10.69 | 33 | 28.90 | 3.09 | 7.73 |
| 13 | 18.73 | 4.74 | 8.44 | 34 | 29.39 | 3.04 | 4.33 |
| 14 | 19.99 | 4.44 | 5.96 | 35 | 29.70 | 3.01 | 7.45 |
| 15 | 20.57 | 4.32 | 2.74 | 36 | 30.52 | 2.93 | 4.19 |
| 16 | 21.08 | 4.21 | 4.51 | 37 | 30.81 | 2.90 | 4.70 |
| 17 | 23.59 | 3.77 | 45.03 | 38 | 31.99 | 2.80 | 2.25 |
| 18 | 24.36 | 3.65 | 4.48 | 39 | 34.29 | 2.61 | 3.87 |
| 19 | 24.89 | 3.58 | 55.05 | 40 | 35.83 | 2.51 | 4.54 |
| 20 | 25.41 | 3.51 | 26.82 | 41 | 39.64 | 2.27 | 4.83 |
| 21 | 25.80 | 3.45 | 22.73 | | | | |

In some embodiments of the present disclosure, a DSC curve of the crystal form B described above has endothermic peaks starting points at 65.1 °C ± 3 °C, 113.7 °C ± 3 °C, 208.8 °C ± 3 °C and 221.1 °C ± 3 °C.

In some embodiments of the present disclosure, a DSC profile of the crystal form B described above is shown in FIG. 7.

In some embodiments of the present disclosure, a TGA curve of the crystal form B described above shows a weight loss of 3.58 ± 0.2% at 150.0 °C ± 3 °C.

In some embodiments of the present disclosure, a TGA profile of the crystal form B described above is shown in FIG. 8.

In another aspect, the present disclosure provides crystal form C of the compound of formula (II), an X-ray powder diffraction pattern of which comprises characteristic peaks at 2Θ values of 7.40 ± 0.20°, 11.21 ± 0.20° and 22.18 ± 0.20°.

In some embodiments of the present disclosure, an X-ray powder diffraction pattern of the crystal form C described above using Cu Kα radiation has diffraction peaks at 2θ values of 7.40 ± 0.20°, 11.21 ± 0.20° and 22.18 ± 0.20°.

In some embodiments of the present disclosure, an X-ray powder diffraction pattern of the crystal form C described above comprises characteristic peaks at 2Θ values of 7.40 ± 0.20°, 11.21 ± 0.20°, 13.95 ± 0.20°, 15.01 ± 0.20°, 15.72 ± 0.20°, 20.61 ± 0.20°, 22.18 ± 0.20° and 23.82 ± 0.20°.

In some embodiments of the present disclosure, an X-ray powder diffraction pattern of the crystal form C described above comprises characteristic peaks at 2Θ values of 7.40° ± 0.20°, 10.50° ± 0.20°, 11.21° ± 0.20°, 11.81° ± 0.20°, 13.05° ± 0.20°, 13.95° ± 0.20°, 15.01° ± 0.20°, 15.72° ± 0.20°, 16.28° ± 0.20°, 17.64° ± 0.20°, 18.35° ± 0.20°, 18.73° ± 0.20°, 19.53° ± 0.20°, 20.14° ± 0.20°, 20.61° ± 0.20°, 22.18° ± 0.20°, 22.51° ± 0.20°, 23.82° ± 0.20°, 24.37° ± 0.20°, 25.49° ± 0.20°, 26.36° ± 0.20°, 27.19° ± 0.20°, 28.93° ± 0.20°, 30.70° ± 0.20°, 31.60° ± 0.20°, 32.50° ± 0.20° and 34.33° ± 0.20°.

In some embodiments of the present disclosure, an X-ray powder diffraction pattern of the crystal form C described above comprises characteristic peaks at 2Θ values of 7.40°, 10.50°, 11.21°, 11.81°, 13.05°, 13.95°, 15.01°, 15.72°, 16.28°, 17.64°, 18.35°, 18.73°, 19.53°, 20.14°, 20.61°, 22.18°, 22.51°, 23.82°, 24.37°, 25.49°, 26.36°, 27.19°, 28.93°, 30.70°, 31.60°, 32.50° and 34.33°.

In some embodiments of the present disclosure, an X-ray powder diffraction pattern of the crystal form C described above is shown in FIG. 3.

In some embodiments of the present disclosure, an X-ray powder diffraction pattern of crystal form C of the compound of formula (II) is shown in Table 3.

**Table 3. XRPD pattern analysis data of crystal form C of the compound of formula (II)**

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 1 | 7.40 | 11.94 | 57.67 | 15 | 20.61 | 4.31 | 29.68 |
| 2 | 10.50 | 8.43 | 9.35 | 16 | 22.18 | 4.01 | 80.86 |
| 3 | 11.21 | 7.90 | 100.00 | 17 | 22.51 | 3.95 | 20.92 |
| 4 | 11.81 | 7.49 | 8.63 | 18 | 23.82 | 3.74 | 39.44 |
| 5 | 13.05 | 6.79 | 3.42 | 19 | 24.37 | 3.65 | 4.57 |
| 6 | 13.95 | 6.35 | 46.42 | 20 | 25.49 | 3.49 | 17.03 |
| 7 | 15.01 | 5.90 | 31.30 | 21 | 26.36 | 3.38 | 3.79 |
| 8 | 15.72 | 5.64 | 38.00 | 22 | 27.19 | 3.28 | 17.79 |
| 9 | 16.28 | 5.45 | 13.70 | 23 | 28.93 | 3.09 | 11.30 |
| 10 | 17.64 | 5.03 | 19.39 | 24 | 30.70 | 2.91 | 3.90 |
| 11 | 18.35 | 4.84 | 12.39 | 25 | 31.60 | 2.83 | 2.44 |
| 12 | 18.73 | 4.74 | 18.09 | 26 | 32.50 | 2.75 | 3.43 |
| 13 | 19.53 | 4.55 | 7.13 | 27 | 34.33 | 2.61 | 3.67 |
| 14 | 20.14 | 4.41 | 2.40 | | | | |

In some embodiments of the present disclosure, a DSC curve of the crystal form C described above has an endothermic peak starting point at 219.9 °C ± 3 °C.

In some embodiments of the present disclosure, a DSC profile of the crystal form C described above is shown in FIG. 9.

In some embodiments of the present disclosure, a TGA curve of the crystal form C described above shows a weight loss of 0.72 ± 0.2% at 170.0 °C ± 3 °C.

In some embodiments of the present disclosure, a TGA profile of the crystal form C described above is shown in FIG. 10.

In some embodiments of the present disclosure, the X-ray powder diffraction pattern is an X-ray powder diffraction pattern of Cu Kα radiation.

In the present disclosure, the compound of formula (I) has the structure shown below:

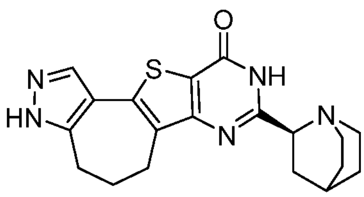

(I)

In yet another aspect, the present disclosure provides a method for preparing a crystal form of the compound of formula (II) described above, the method comprising the following steps:

(1) mixing a compound of formula (I) as shown below with methanol;
(2) adding maleic acid to the mixture of step (1);
(3) performing filtration and drying to obtain the crystal form A or crystal form C described above; and
(4) mixing the crystal form A described above with acetonitrile and water, and after the solid is dissolved, volatilizing the solvents completely to obtain the crystal form B described above,

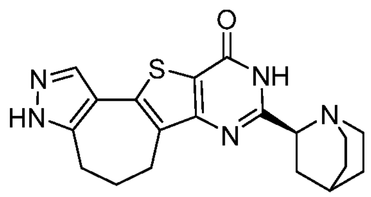

(I) .

In some embodiments of the present disclosure, the method for preparing a crystal form of the compound of formula (II) described above is a method for preparing the crystal form A described above, the method comprising the following steps:

(1) mixing the compound of formula (I) described above with methanol at 20-30 °C;
(2) cooling the mixture of step (1) to 5 °C with stirring, then adding maleic acid to the mixture, and stirring the mixture at 5-13 °C for 6 h; and
(3) performing filtration and drying to obtain the crystal form A described above.

In some embodiments of the present disclosure, the method for preparing a crystal form of the compound of formula (II) described above is a method for preparing the crystal form B described above, the method comprising the following steps: mixing the crystal form A described above with acetonitrile and water, and after the solid is dissolved, volatilizing the solvents completely at 40 °C to obtain the crystal form B described above.

In some embodiments of the present disclosure, the method for preparing a crystal form of the compound of formula (II) described above is a method for preparing the crystal form C described above, the method comprising the following steps:

(1) mixing the compound of formula (I) described above with methanol at 20-30 °C;
(2) adding maleic acid to the mixture of step (1) with stirring at 75 °C and stirring the mixture at 75 °C for 0.5 h, stirring the mixture at 40 °C for 1 h, cooling the mixture to 25 °C, and stirring the mixture for another 2 h;
(3) performing filtration and drying to obtain the crystal form C described above.

In yet another aspect, the present disclosure provides a crystalline composition comprising crystal form A of the compound of formula (II), crystal form B of the compound of formula (II) or crystal form C of the compound of formula (II), wherein crystal form A of the compound of formula (II), crystal form B of the compound of formula (II) or crystal form C of the compound of formula (II) makes up 50% or more, preferably 75% or more, more preferably 90% or more, and most preferably 95% or more by weight of the crystalline composition. The crystalline composition may also contain a small amount of the compound of formula (II) in other crystal forms or amorphous form.

In yet another aspect, the present disclosure provides a pharmaceutical composition comprising the compound described above or the crystal form described above, and optionally a pharmaceutically acceptable excipient.

In another aspect, the present disclosure also provides use of the compound described above or the crystal form described above or the pharmaceutical composition described above for preparing a Cdc7 inhibitor.

The present disclosure also provides use of the compound described above or the crystal form described above or the crystalline composition described above or the pharmaceutical composition described above for preparing a medicament for preventing or treating a Cdc7 kinase-mediated disease (e.g., a tumor).

The present disclosure also provides a method for preventing or treating a Cdc7 kinase-mediated disease comprising administering to a mammal, preferably a human, in need thereof a therapeutically effective amount of the compound described above or the crystal form described above or the crystalline composition described above or the pharmaceutical composition described above.

The present disclosure also provides use of the compound described above or the crystal form described above or the crystalline composition described above or the pharmaceutical composition described above in preventing or treating a Cdc7 kinase-mediated disease.

The present disclosure also provides the compound described above or the crystal form described above or the crystalline composition described above or the pharmaceutical composition described above for use in preventing or treating a Cdc7 kinase-mediated disease.

The present disclosure also provides the compound described above or the crystal form described above or the crystalline composition described above or the pharmaceutical composition described above for use as a Cdc7 inhibitor.

The present disclosure also provides the compound described above or the crystal form described above or the crystalline composition described above or the pharmaceutical composition described above for use as a medicament for preventing or treating a Cdc7 kinase-mediated disease.

In some embodiments of the present disclosure, the Cdc7 kinase-mediated disease is a tumor, e.g., colorectal cancer or pancreatic cancer.

In some embodiments of the present disclosure, the Cdc7 inhibitor is a medicament for treating a tumor, e.g., a medicament for treating colorectal cancer or pancreatic cancer.

**Technical Effects**

The processes of preparing the compound and the crystal forms disclosed herein are simple, and the crystal forms are relatively stable and less affected by heat and humidity, which favors the preparation of preparations. The compound and the crystal forms thereof disclosed herein have good inhibitory activity against Cdc7: they have relatively strong inhibitory effects on enzyme CDC7/DBF4 and have inhibitory effects on the proliferation of colorectal cell COLO205 that highly expresses Cdc7. Therefore, the compound and the crystal forms disclosed herein are effective novel drugs having the potential for use in clinically treating tumors.

The crystal forms disclosed herein have advantages in such aspects as pharmaceutical activity, hygroscopicity, stability, purity, and easiness of preparation, so they can meet the requirements of the production, storage, transport, preparation, etc. of drugs.

**Definitions and Description**

Unless otherwise stated, the following terms and phrases used herein are intended to have the following meanings. A particular phrase or term, unless otherwise specifically defined, should not be considered as uncertain or unclear, but construed according to its common meaning. When referring to a trade name, it is intended to refer to its corresponding commercial product or its active ingredient.

The word "comprise", and variants thereof such as "comprises" or "comprising", or equivalents shall be understood in an open, non-exclusive sense, i.e., "includes but is not limited to", indicating that in addition to the listed elements, components and procedures, other unspecified elements, components and procedures may also be encompassed.

The compound disclosed herein may demonstrate a specific geometric isomerism or stereoisomerism. All such compounds are contemplated herein, including *cis* and *trans* isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereomer enriched mixture, all of which are encompassed within the scope of the present invention. Substituents such as alkyl may have an additional asymmetric carbon atom. All these isomers and mixtures thereof are encompassed within the scope of the present invention.

Unless otherwise stated, "(*D*)" or "(+)" stands for dextrorotation, "(*L*)" or "(-)" stands for levorotation, and "(*DL*)" or "(±)" stands for racemization.

Unless otherwise stated, the absolute configuration of a stereogenic center is represented by a wedged solid bond (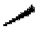) and a wedged dashed bond (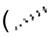), and the relative configuration of a stereogenic center is represented by a straight solid bond (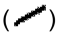) and a straight dashed bond (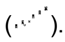). A wavy line (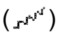) represents a wedged solid bond (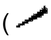) or a wedged dashed bond (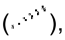), or a wavy line (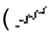) represents a straight solid bond (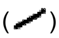) and a straight dashed bond (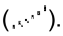).

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio. The term "pharmaceutically acceptable excipient" refers to an inert substance administered with an active ingredient to facilitate administration of the active ingredient, including but not limited to, any glidant, sweetener, diluent, preservative, dye/coloring agent, flavor enhancer, surfactant, wetting agent, dispersant, disintegrant, suspending agent, stabilizer, isotonizing agent, solvent or emulsifier acceptable for use in humans or animals (e.g., domesticated animals) as permitted by the National Medical Products Administration, PRC. Non-limiting examples of the excipients include calcium carbonate, calcium phosphate, various sugars and types of starch, cellulose derivatives, gelatin, vegetable oils, and polyethylene glycols.

The term "pharmaceutical composition" refers to a mixture consisting of one or more of the compounds or the crystal forms thereof disclosed herein and a pharmaceutically acceptable excipient. The pharmaceutical composition is intended to facilitate the administration of the compound disclosed herein to an organism.

The pharmaceutical composition disclosed herein can be prepared by combining the compound or the crystal

forms thereof disclosed herein with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere and aerosol.

Typical routes of administration of the compound or the crystal forms thereof or the pharmaceutical composition thereof disclosed herein include, but are not limited to, oral, rectal, topical, inhalation, parenteral, sublingual, intravaginal, intranasal, intraocular, intraperitoneal, intramuscular, subcutaneous and intravenous administration. The pharmaceutical composition disclosed herein can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound disclosed herein to be formulated into tablets, pills, pastilles, dragees, capsules, liquids, gels, slurries, suspensions, etc. for oral administration to a patient.

Therapeutic dosages of the compound or the crystal forms thereof disclosed herein may be determined by, for example, the specific use of a treatment, the route of administration of the compound, the health and condition of a patient, and the judgment of a prescribing physician. The proportion or concentration of the compound or the crystal form disclosed herein in the pharmaceutical composition may vary depending on a variety of factors including dosages, chemical properties (e.g., hydrophobicity), and routes of administration.

The term "treating" refers to administering the compound or the crystal form thereof or the pharmaceutical composition thereof disclosed herein to ameliorate or eliminate a disease or one or more symptoms associated with the disease, and includes:

(i) inhibiting a disease or disease state, i.e., arresting its development; and
(ii) alleviating a disease or disease state, i.e., causing its regression.

The term "prevent", "preventing" or "prevention" refers to administering the compound or the crystal form thereof or the pharmaceutical composition thereof disclosed herein so as to prevent a disease or one or more symptoms associated with the disease, and includes: preventing the occurrence of the disease or disease state in a mammal, particularly when such a mammal is predisposed to the disease state but has not yet been diagnosed as having it.

"Mammal" includes humans, domestic animals such as laboratory mammals and domestic pets (e.g., cat, dog, pig, cow, sheep, goat, horse, rabbit), and non-domesticated mammals such as wild mammals.

For drugs and pharmacologically active agents, the term "therapeutically effective amount" refers to an amount of a drug or a medicament that is sufficient to provide the desired effect and is non-toxic. The determination of the effective amount varies from person to person. It depends on the age and general condition of a subject, as well as the particular active substance used. The appropriate effective amount in a case may be determined by those skilled in the art in the light of conventional tests.

Unless otherwise specified clearly herein, singular terms encompass plural terms, and vice versa.

Unless otherwise stated herein, parameter values (including 2Θ values and reaction conditions) are to be construed as modified by the term "about" to reflect the measurement error and the like existing in the values, e.g., there is an error of ±5% relative to the given value.

All patents, patent applications and other identified publications are explicitly incorporated herein by reference for the purpose of description and disclosure. These publications are provided solely because they were disclosed prior to the filing date of the present disclosure. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

The intermediate compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure.

The compound disclosed herein may be structurally confirmed by conventional methods well known to those skilled in the art; if the present disclosure relates to the absolute configuration of the compound, this absolute configuration may be confirmed by means of conventional techniques in the art. For example, in the single crystal X-ray diffraction (SXRD) method, intensity data of diffraction of the single crystal grown are collected with a Bruker D8 venture diffractometer, the light source is CuKα radiation, and the scan mode is φ/ scan; after related data are collected, the direct method (Shelxs97) is further employed to analyze the crystal structure, and thus the absolute configuration can be confirmed.

The chemical reactions of the embodiments of the present disclosure are conducted in a proper solvent that

must be suitable for the chemical changes in the present disclosure and the reagents and materials required. In order to acquire the compound disclosed herein, it is sometimes necessary for those skilled in the art to modify or select a synthesis procedure or a reaction process based on the existing embodiments.

The present disclosure is described in detail below by way of examples, which are not intended to limit the present disclosure in any way.

All the solvents used in the present disclosure are commercially available and can be used without further purification.

The solvents used in the present disclosure are commercially available.

The present disclosure uses the following abbreviations: DMF-DMA: N,N-dimethylformamide dimethyl acetal; DHP: 3,4-dihydropyran; DMF: N,N-dimethylformamide; MeOH: methanol; THF: tetrahydrofuran.

It should be noted that in an X-ray powder diffraction pattern, the position and relative intensity of a peak may vary depending on the measuring instrument, the measuring method/conditions, and other factors. For any particular crystal form, the position of a peak may have an error, and the measurement of 2Θ may have an error of ±0.2°. Therefore, this error should be considered when determining each crystal form, and crystal forms within this margin of error are within the scope of the present disclosure.

It should be noted that, for the same crystal form, the position of an endothermic peak in the DSC (differential scanning calorimetry) pattern may vary depending on the measuring instrument, the measuring method/conditions, and other factors. For any particular crystal form, the position of an endothermic peak may have an error of ±5 °C or ±3 °C. Therefore, this error should be considered when determining each crystal form, and crystal forms within this margin of error are within the scope of the present disclosure.

It should be noted that, for the same crystal form, the temperature and value of a weight loss in the TGA pattern may vary depending on the measuring instrument, the measuring method/conditions, and other factors. For any particular crystal form, the temperature and value of a weight loss may have errors: the weight loss temperature may have an error of ±5 °C and the weight loss value may have an error of ±0.2%. Therefore, this error should be considered when determining each crystal form, and crystal forms within this margin of error are within the scope of the present disclosure.

It should be noted that in an infrared spectrum, the position of a peak may vary depending on the measuring instrument, the measuring method/conditions, and other factors. For any particular crystal form, the position of a peak may have an error: the position of a peak may have a measurement error of ±5 $cm^{-1}$. Therefore, this error should be considered when determining each crystal form, and crystal forms within this margin of error are within the scope of the present disclosure.

It should be noted that in an ultraviolet absorption spectrum, the position of a peak may vary depending on the measuring instrument, the measuring method/conditions, and other factors. For any particular crystal form, the position of a peak may have an error, and the wavelength may have a measurement error of ±5 nm or ±3 nm. Therefore, this error should be considered when determining each crystal form, and crystal forms within this margin of error are within the scope of the present disclosure.

Unless otherwise stated herein, "room temperature" in the present disclosure refers to normal temperature, typically 20-30 °C.

## Instruments and analytical methods

### 1.1. X-ray powder diffraction (X-ray powder diffractometer, XRPD)

Instrument model: X'Pert3 X-ray diffractometer from PANalytical Inc.

Method: take about 10mg sample for XRPD analysis.

The XRPD parameters are detailed below:

X-ray tube: Cu, ka (λ = 1.54060 **Å**)

Voltage of X-ray tube: 45 kV, current of X-ray tube: 40 mA

Divergent slit: 0.60 mm

Detector slit: 10.50 mm

Anti-scatter slit: 7.10 mm

Scan range: 3-40 deg

Step size: 0.0263 deg

Step time: 0.12 s

Rotational speed of sample tray: 15 rpm

**1.2. Differential scanning calorimetry (differential scanning calorimeter, DSC)**

Instrument model: TA 2500 differential scanning calorimeter
Method: take a sample (0.5-1 mg) and put it into a DSC aluminum pan for analysis: under 50 mL/min $N_2$, heat the sample from room temperature (25 °C) to 300 °C or 350 °C at a rate of 10 °C/min.

**1.3. Thermogravimetric analysis (thermal gravimetric analyzer, TGA)**

Instrument model: TA Q5500/Q5000 thermogravimetric analyzer
Method: take a sample (2-5 mg) and put it into a TGA platinum pan for analysis: under 25 mL/min $N_2$, heat the sample from room temperature (25 °C) to 300 °C or 350 °C at a rate of 10 °C/min to make a weight loss of 20%.

**1.4. Judgment criteria for hygroscopicity:**

**Table 4. Judgment criteria for hygroscopicity**

| Categories of hygroscopicity | Hygroscopic weight gain* |
|---|---|
| Deliquescence | Absorb sufficient water to form a solution |
| Very hygroscopic | Hygroscopic weight gain ≥ 15% |
| Moderately hygroscopic | 2% ≤ Hygroscopic weight gain < 15% |
| Slightly hygroscopic | 0.2% ≤ Hygroscopic weight gain < 2% |
| Non-hygroscopic or hardly hygroscopic | Hygroscopic weight gain < 0.2% |
| *Hygroscopic weight gain at 25 °C/80% RH. | |

**1.5. High performance liquid chromatography (high performance liquid chromatograph, HPLC)**

Instrument model: Agilent 1200 high performance liquid chromatograph
The analytical method is as follows:

**Table 5. The HPLC analytical method for determining the content of related substances**

| | |
|---|---|
| Chromatography column | Waters XBridge Shield RP18, 4.6×150mm, 3.5μm |
| Column temperature | 40 °C |
| Flow rate | 1.0 mL/min |
| Detector | DAD |
| Detection wavelength | 220 nm |
| Injection volume | 5 μL |
| MPA | 0.1%TFA in $H_2O$, V/V |
| MPB | 0.05%TFA in ACN, V/V |
| Needle wash solution | ACN:$H_2O$=1:1, V/V |

(continued)

| Diluent | Acetonitrile:0.1% aqueous trifluoroacetic acid = 20:80, V/V | | |
|---|---|---|---|
| Elution program | Time (min) | A (%) | B (%) |
| | 0 | 100 | 0 |
| | 5 | 100 | 0 |
| | 25 | 20 | 80 |
| | 30 | 20 | 80 |
| | 31 | 100 | 0 |
| | 40 | 100 | 0 |

## BRIEF DESCRIPTION OF THE DRAWINGS

FIG. 1 shows an XRPD pattern of crystal form A of the compound of formula (II).
FIG. 2 shows an XRPD pattern of crystal form B of the compound of formula (II).
FIG. 3 shows an XRPD pattern of crystal form C of the compound of formula (II).
FIG. 4 shows a DSC profile of crystal form A of the compound of formula (II).
FIG. 5 shows a TGA profile of crystal form A of the compound of formula (II).
FIG. 6 shows an infrared absorption spectrum of crystal form A of the compound of formula (II).
FIG. 7 shows a DSC profile of crystal form B of the compound of formula (II).
FIG. 8 shows a TGA profile of crystal form B of the compound of formula (II).
FIG. 9 shows a DSC profile of crystal form C of the compound of formula (II).
FIG. 10 shows a TGA profile of crystal form C of the compound of formula (II).
FIG. 11 shows an ultraviolet absorption spectrum of crystal form A of the compound of formula (II).
FIG. 12 shows an ellipsoid plot of the three-dimensional structure of the compound of formula (I).

## DETAILED DESCRIPTION

The present disclosure is described in detail below by way of examples. However, this is by no means disadvantageously limiting the scope of the present disclosure. Although the present disclosure has been described in detail herein and specific embodiments have also been disclosed, it will be apparent to those skilled in the art that various changes and modifications can be made to the specific embodiments without departing from the spirit and scope of the present disclosure.

### Example 1: Compounds of Formula (I)

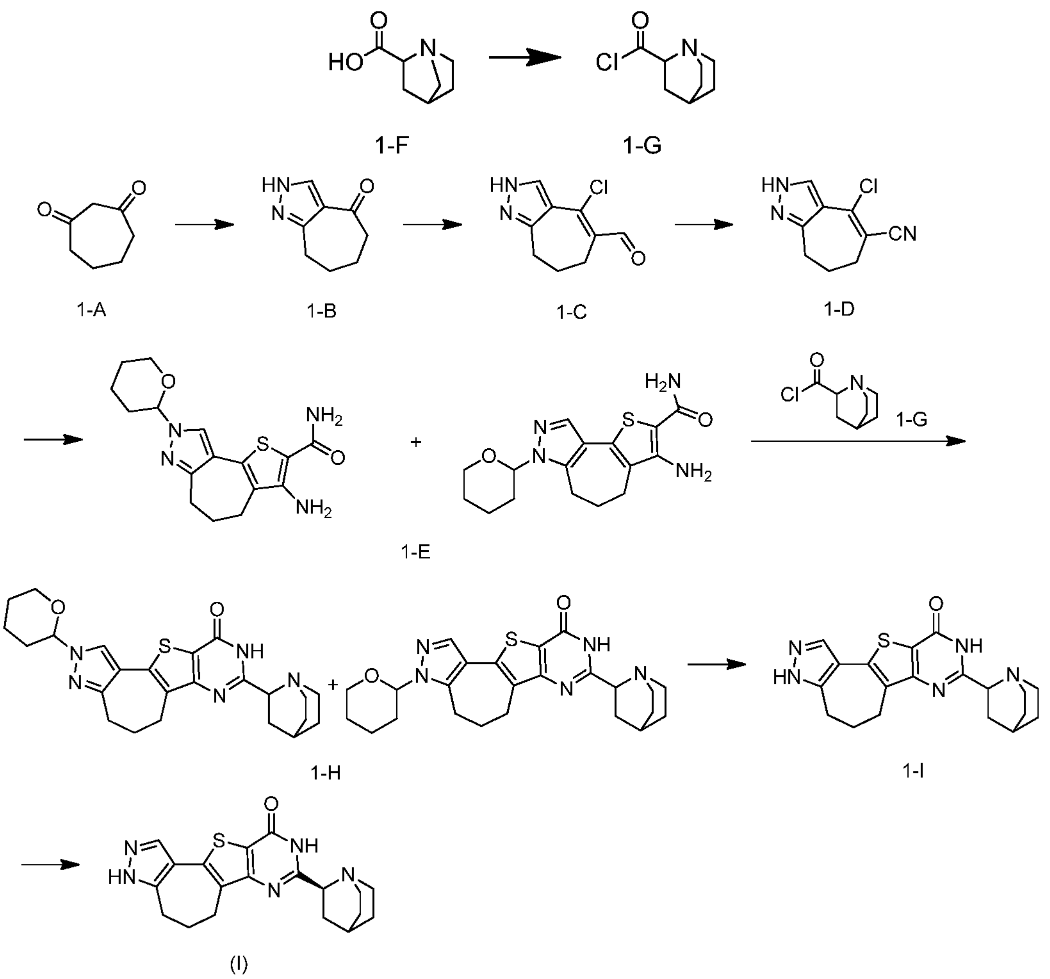


1-F 1-G

1-A 1-B 1-C 1-D

1-E

1-H 1-I

(I)

Step 1: Synthesis of intermediate 1-B

DMF-DMA (19.05 mol, 1.0 *eq,* 2.53 L) was added to a 30 L high-low temperature reaction kettle at room temperature, and starting material 1-A (2403.52 g, 19.05 mol, 1.0 *eq)* was added in portions with stirring. The reaction was slightly exothermic, so the rate of dropwise addition was controlled to ensure that the internal temperature of the reaction mixture did not exceed 35 °C. After the addition, the reaction mixture was heated to 85-90 °C, stirred at that temperature for 3 h, and cooled to 50-55 °C. Methanol (2.4 L) was added as a solvent, and then hydrazine hydrate (17.15 mol, 98% purity, 0.9 *eq,* 850 mL) was slowly added dropwise. The reaction was exothermic, so the rate of addition was controlled to prevent bumping. After the dropwise addition, the mixture was reacted at 50-60 °C for 0.5 h. The reaction mixture was cooled to room temperature and concentrated at 50 °C under a reduced pressure of -0.08 MPa. The resulting solid was triturated with ethyl acetate (2.4 L). After 0.5 h of stirring, the mixture was filtered, and the filter cake was collected. The filtrate was concentrated under reduced pressure to give a crude product. The crude product was triturated with ethyl acetate (1.2 L). After 0.5 h of stirring, the mixture was filtered, and the filter cake was collected. The filter cakes were combined and dried *in vacuo* at 45-50 °C under -0.08 MPa to constant weight to give 1-B. LCMS (ESI) m/z: 151 (M+1); $^1$H NMR (DMSO-$d_6$, 400 MHz) δ ppm 13.02 (s, 1H), 7.82 (s, 1H), 2.91-2.96 (m, 2H), 2.56-2.61 (m, 2H), 1.85-1.91 (m, 2H), 1.78-1.84 (m, 2H).

Step 2: Synthesis of intermediate 1-C

Dichloromethane (11.07 L) was added as a solvent to a 50 L high-low temperature reaction kettle at room temperature, and intermediate 1-B (2214.90 g, 14.74 mol, 1.0 *eq)* and N,N-dimethylformamide (3.40 L, 44.23 mol, 3.0 *eq)* were added in portions with stirring. Oxalyl chloride (3.87 L, 44.23 mol, 3.0 *eq)* was then slowly added dropwise, and the rate of addition was controlled so the internal temperature of the reaction mixture did not exceed 30 °C. The reaction mixture was stirred at 20-30 °C for 12 h and concentrated to dryness at 40 °C under a reduced pressure of -0.08 MPa. The resulting crude product was added in portions to ice water (22.14 L) with stirring at room temperature.

After 0.5 h of stirring, the mixture was filtered, and the filter cake was collected, washed with water (2 L × 3), and dried *in vacuo* at 35-40 °C under -0.08 MPa to give 1-C. LCMS (ESI) m/z: 197 (M+1); $^1H$ NMR (DMSO-$d_6$, 400 MHz) δ ppm 10.2 (s, 1H), 8.03 (s, 1H), 2.9-3.0 (m, 2H), 2.6-2.6 (m, 2H), 1.8-1.8 (m, 2H).

Step 3: Synthesis of intermediate 1-D

THF (14.65 L) was added as a solvent to a 50 L high-low temperature reaction kettle at room temperature, and intermediate 1-C (2929.34 g, 14.84 mol, 1.0 *eq)* and concentrated ammonium hydroxide (4.57 L, 29.69 mol, 2.0 *eq)* were added in portions with stirring. Elemental iodine (7540.23 g, 29.69 mol, 2.0 *eq)* was slowly added in portions, and the rate of addition was controlled so the internal temperature of the reaction mixture did not exceed 35 °C. The reaction mixture was stirred at 20-35 °C for 12 h and added to 120 L of saturated aqueous sodium sulfite solution with stirring. After 0.5 h of stirring, the mixture was filtered, and the filter cake was collected, washed with water (3 L × 3), and dried *in vacuo* at 45-50 °C under -0.08 MPa to constant weight to give 1-D. LCMS (ESI) m/z: 194 (M+1); $^1H$ NMR (DMSO-$d_6$, 400 MHz) δ ppm 13.06-13.27 (m, 1H), 7.71-8.11 (m, 1H), 2.90-2.96 (m, 2H), 2.67 (m, 2H), 1.90 (m, 2H).

Step 4: Synthesis of intermediate 1-E

THF (9.70 L) was added to a 50 L high-low temperature reaction kettle at room temperature, and intermediate 1-D (1.94 kg, 9.82 mol, 98.061% purity, 1 *eq)* and DHP (2.48 kg, 29.47 mol, 2.69 L, 3.0 *eq,* 1000 mL/min) were then added in sequence to the reaction kettle. The reaction mixture was stirred at 75-80 °C for 65 h and concentrated at 50 °C under a reduced pressure of -0.08 MPa to give an oil. DMF (15.90 L) was added as a solvent to a 50 L high-low temperature reaction kettle at room temperature, and the oil obtained above (3180.26 g, 11.45 mol, 1 *eq),* mercaptoacetamide (1252.14 g, 13.74 mol, 1.2 *eq)* and potassium carbonate (3323.28 kg, 24.05 mol, 2.1 *eq)* were sequentially added in portions to the reaction kettle as starting materials. The reaction was not exothermic. The mixture was reacted at 75-80 °C for 1 h (the passages of the system were kept clear, a small flow of nitrogen was maintained and constant stirring was ensured; gaseous waste should be absorbed with a sodium hypochlorite solution, and a buffer safety bottle was arranged between the gaseous waste absorption setup and the reaction kettle) and cooled to room temperature, and ethyl acetate (15.90 L) was added. The reaction mixture was slowly poured into 160 L of water with stirring. After the addition, a suspension was obtained. The suspension was stirred at room temperature for 12 h and then filtered (solid precipitation was slow), and the filter cake was collected, washed with water (3 L × 3), and added to 15.90 L of methanol. The mixture was stirred at 65-70 °C for 1 h, cooled to room temperature, and then filtered, and the filter cake was collected (if it was not pure enough, the above purification process may be repeated) and dried *in vacuo* at 45-50 °C under -0.08 MPa to give 1-E. LCMS (ESI) m/z: 333 (M+1); $^1H$ NMR (DMSO-$d_6$, 400 MHz) δ ppm 7.52-7.92 (m, 1H), 6.62-6.89 (m, 2H), 6.29-6.50 (m, 2H), 5.22-5.52 (m, 1H), 3.83-3.99 (m, 1H), 3.55-3.70 (m, 1H), 2.84-3.18 (m, 2H), 2.60-2.71 (m, 2H), 1.86-2.35 (m, 5H), 1.43-1.75 (m, 3H).

Step 5: Synthesis of intermediate 1-G

Starting material 1-F (906.50 g, 4.73 mol, 1 *eq,* HCl) was added to dichloromethane (9.6 L) at room temperature, and oxalyl chloride (331.13 g, 2.61 mol, 228.37 mL, 5 *eq)* was added dropwise at 10-15 °C. The mixture was then stirred for 72 h and concentrated to dryness under reduced pressure. 5 L of DCM was added and the mixture was concentrated under reduced pressure to remove the residual oxalyl chloride; the process was repeated once. The resulting 1-G was directly used in the next step.

Step 6: Synthesis of intermediate 1-H

9.2 L of dichloromethane was added to a 30 L high-low temperature reaction kettle at room temperature, and 1-G (983.28 g, 4.68 mol, 1.7 *eq,* HCl) was added to the reaction kettle with stirring. The mixture was cooled to -20 to -15 °C. 1-E (920.19 g, 2.77 mol, 1 *eq)* was added in portions to the reaction kettle, and the internal temperature was controlled at below -10 °C. The mixture was stirred at -20 to -10 °C for 0.5 h. HPLC analysis showed the 1-E content < 5%. Triethylamine (192 mL, 1.39 mol, 0.5 *eq)* was added dropwise to the reaction kettle at -20 to -10 °C. A sample was then taken, and HPLC analysis showed the 1-E content < 1%. The remaining triethylamine (960 mL, 6.93 mol, 2.5 *eq)* was added dropwise to the reaction kettle. The mixture was concentrated under reduced pressure, and the resulting solid (about 3 kg) was triturated with 15 L of n-heptane to remove dichloromethane. The mixture was filtered, and the solid was collected and dried under reduced pressure (45 °C, -0.1 MPa) to give 1-H-M.

8.7 L of methanol and 4.3 L of water (methanol:water = 2:1) were added to a high-low temperature reaction kettle, and after sodium hydroxide (1107.27 g, 27.7 mol, 10 *eq)* was added, the temperature was raised to 50 °C. The dried 1-H-M was added in portions to the reaction kettle. After the mixture was stirred for 0.5 h, HPLC analysis showed

the reaction was complete. The mixture was cooled to 0 °C, and 13 L of water was added. The mixture was stirred at 0-5 °C for 0.5 h and filtered, and the solid was collected. The filtrate was adjusted to pH 7-8 with 6 N hydrochloric acid and concentrated under reduced pressure to remove most methanol. The pH was adjusted to 10-11 with 2 N sodium hydroxide at 10-15 °C, and a large amount of solid precipitated. The mixture was stirred for another 0.5 h and filtered, and the filter cake was collected. The filter cakes were combined and washed with water (4 L × 2). After filtration, the filter cake was dried under reduced pressure (55 °C and -0.08 MPa) to give 1-H. LCMS (ESI) m/z: 452 (M+1); $^1$H NMR (DMSO-$d_6$+$D_2O$, 400 MHz) δ ppm 7.66-8.08 (m, 1H), 5.30-5.43 (m, 1H), 3.85-4.00 (m, 1H), 3.55-3.76 (m, 3H), 2.89-3.12 (m, 6H), 2.74-2.87 (m, 1H), 2.37-2.45 (m, 1H), 2.05-2.22 (m, 1H), 1.86-2.05 (m, 4H), 1.76-1.84 (m, 1H), 1.61-1.75 (m, 2H), 1.60 (br s, 5H), 1.28-1.41 (m, 1H).

Step 7: Synthesis of intermediate 1-I

1-H (1197.21 g, 2.65 mol, 1 *eq)* was added in portions to a 4 N solution of hydrogen chloride in ethyl acetate (11.97 L, 47.88 mol, 18.1 eq) with stirring at 20-25 °C. After the addition, the mixture was stirred at 10-15 °C for 0.5 h and then filtered, and the filter cake was washed with ethyl acetate (1 L × 2) and dried under reduced pressure (40-45 °C, -0.1 MPa) for 16 h to give 1-I-M. 1-I-M was added to a mixed solvent of water (9.6 L) and methanol (1.2 L), and the pH was adjusted to 8-9 with concentrated ammonium hydroxide. The mixture was stirred for 0.5 h and filtered, and the filter cake was collected and washed with water (5 L × 2). After filtration, the filter cake was collected and dried under reduced pressure (55 °C, -0.1 MPa) to give 1-I. LCMS (ESI) m/z: 368 (M+1); $^1$H NMR (DMSO-$d_6$+$D_2O$, 400 MHz) δ ppm 7.46 (s, 1H), 3.64-3.78 (m, 1H), 2.89-3.03 (m, 5H), 2.72-2.89 (m, 3H), 2.30-2.36 (m, 1H), 1.73-1.88 (m, 4H), 1.35-1.54 (m, 4H).

Step 8: Synthesis of the compounds of formula (I)

First resolution:

10.1 L of methanol was added to a 30 L high-low temperature reaction kettle at room temperature, and 1-I (807.89 g, 2.20 mol, 1 *eq)* was added to the reaction kettle. Glacial acetic acid (4 L) and water (404 mL) were added in sequence with stirring. When the temperature of the reactor reached 60 °C, 3.7 L of methanol in which (+)-di-p-toluoyl-D-tartaric acid (374.59 g, 0.97 mmol, 0.44 eq) was dissolved was added in portions to the reactor. The mixture was stirred at 60-65 °C for 12 h, cooled to 20 °C at a rate of 10 °C/30 min, and then filtered, and the filter cake was washed with methanol (4.6 L). After filtration, the filter cake was dispersed in a mixed solvent of water (8 L) and methanol (1 L). The mixture was adjusted to pH 8-9 with concentrated ammonium hydroxide, stirred for 0.5 h, and filtered. The filter cake was collected and washed twice with water by trituration. After filtration, the filter cake was collected and dried under reduced pressure (55 °C, -0.1 MPa) to give 1-J-M1 (89.116% ee).

Second resolution:

3.78 L of methanol and 1-J-M1 (302.67 g, 0.824 mol, 1 *eq)* were added to a 10 L high-low temperature reaction kettle at room temperature. Glacial acetic acid (1.5 L) and water (151 mL) were added with stirring. When the temperature of the reactor reached 60 °C, 2.74 L of methanol in which (+)-di-p-toluoyl-D-tartaric acid (273.67 g, 0.708 mmol, 0.86 *eq)* was dissolved was added in one portion to the reactor. The reaction mixture was stirred at 60-65 °C for 12 h under a small flow of nitrogen, cooled to room temperature at a rate of 10 °C/30 min, and then filtered, and the filter cake was triturated with methanol (3.2 L). After filtration, the filter cake was dispersed in a mixed solvent of water (8.52 L) and methanol (1.07 L). The mixture was adjusted to pH 8-9 with concentrated ammonium hydroxide, stirred for 0.5 h, and then filtered. The filter cake was collected and washed twice with water. After filtration, the filter cake was collected and dried under reduced pressure (55 °C, -0.1 MPa) to give 1-J-M2 (98.040% ee).

Third resolution:

3.14 L of methanol was added to a 10 L high-low temperature reaction kettle at room temperature, and 1-J-M2 (251.36 g, 0.68 mol, 1 *eq)* was added to the reaction kettle. Glacial acetic acid (1.25 L) and water (125 mL) were added in sequence with stirring. When the temperature of the reactor reached 60 °C, 2.39 L of methanol in which (+)-di-p-toluoyl-D-tartaric acid (239.22 g, 0.62 mmol, 0.91 eq) was dissolved was added in one portion to the reactor. The reaction mixture was stirred at 60-65 °C for 12 h under a small flow of nitrogen, cooled to room temperature at a rate of 10 °C/30 min, and then filtered, and the filter cake was triturated with methanol (3 L). After filtration, the filter cake was dispersed in a mixed solvent of water (4.8 L) and methanol (0.6 L). The mixture was adjusted to pH 8-9 with concentrated ammonium hydroxide, stirred for 0.5 h, and filtered. The filter cake was collected and washed twice with water by trituration. After

filtration, the filter cake was collected and dried under reduced pressure (55 °C, -0.1 MPa) to give the compound of formula (I) (99.76% ee). LCMS (ESI) m/z: 368 (M+1); $^1H$ NMR (DMSO-$d_6$, 400 MHz): δ (ppm) 7.92 (m, 1H), 3.86-3.92 (m, 1H), 3.03-3.13 (m, 5H), 2.82-2.92 (m, 1H), 2.55-2.67 (m, 2H), 2.29-2.39 (m, 1H), 1.93-2.05 (m, 2H), 1.83-1.92 (m, 1H), 1.70-1.81 (m, 1H), 1.50-1.62 (m, 2H), 1.37-1.49 (m, 2H).

**Example 2: Crystal Form A of Compound of Formula (II)**

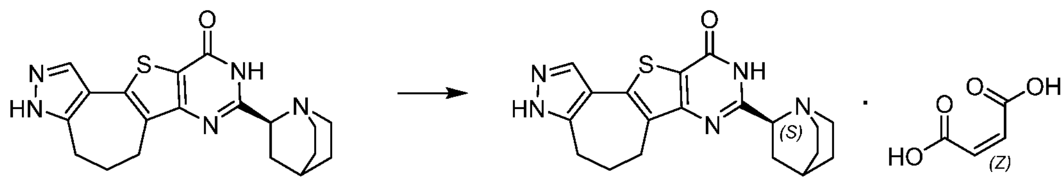

(I) (II)

Methanol (1042 mL) was added to a 3 L reaction flask at room temperature, and the compound of formula (I) (45.00 g, 122.46 mmol, 1 *eq)* was then added to the reaction flask. The mixture was cooled to 5 °C with stirring, and maleic acid (21.32 g, 183.69 mol, 1.5 *eq)* was added thereto. The mixture was stirred at 5-13 °C for 6 h and filtered, and the filter cake was collected, washed with methanol (500 mL × 2), and then dried under reduced pressure (45 °C, -0.1 MPa) to give crystal form A of the compound of formula (II). LCMS (ESI) m/z: 368 (M+1); $^1H$ NMR (heavy water, 400 MHz): δ ppm 7.46 (s, 1H), 6.16 (s, 2H), 4.45-4.55 (m, 1H), 3.64-3.81 (m, 1H), 3.48-3.59 (m, 1H), 3.34-3.47 (m, 2H), 2.76-2.87 (m, 2H), 2.65-2.76 (m, 2H), 2.41-2.53 (m, 1H), 2.26-2.37 (m, 1H), 1.95-2.09 (m, 3H), 1.85-1.95 (m, 2H), 1.63-1.84 (m, 2H).

$^1H$ NMR analysis shows that the salt formation number of crystal form A of the compound of formula (II) is 1.

**Example 3: Crystal Form B of Compound of Formula (II)**

Crystal form A of the compound of formula (II) (60.91 mg) was measured out and added to a mixture of acetonitrile (1 mL) and water (1 mL), and the solid was dissolved by stirring. The solvent was completely volatilized at 40 °C, and a solid precipitated. The solid was collected and dried under reduced pressure (55 °C, -0.1 MPa) to give crystal form B of the compound of formula (II). LCMS (ESI) m/z: 368 (M+1); $^1H$ NMR (DMSO-$d_6$+$D_2O$, 400 MHz): δ ppm 7.85 (s, 1H), 6.10 (s, 2H), 4.60-4.64 (m, 1H), 3.74-3.86 (m, 1H), 3.38-3.52 (m, 1H), 3.21-3.37 (m, 2H), 3.04 (br s, 4H), 2.37-2.47 (m, 1H), 2.16-2.24 (m, 1H), 2.02-2.12 (m, 1H), 1.91-2.01 (m, 2H), 1.87 (br s, 4H).

$^1H$ NMR analysis shows that the salt formation number of crystal form B of the compound of formula (II) is 1.

**Example 4: Crystal Form C of Compound of Formula (II)**

Methanol (56 mL) was added to the compound of formula (I) (0.80137 g, 2.18 mmol, 1 *eq)* at room temperature, and maleic acid (505.29 mg, 4.35 mmol, 2 *eq)* was added with stirring at 75 °C. The mixture was stirred at 75 °C for 0.5 h and at 40 °C for 1 h, cooled to 25 °C, stirred for another 2 h, and filtered. The solid was collected, washed with methanol (10 mL × 2), and dried under reduced pressure (55 °C, -0.1 MPa) to give crystal form C of the compound of formula (II). LCMS (ESI) m/z: 368 (M+1); $^1H$ NMR (DMSO-$d_6$+$D_2O$, 400 MHz): δ ppm 7.92 (s, 1H), 6.09 (s, 2H), 4.62-4.64 (m, 1H), 3.62-3.75 (m, 1H), 3.39-3.54 (m, 1H), 3.23-3.37 (m, 2H), 3.04 (br s, 4H), 2.37-2.48 (m, 1H), 2.15-2.28 (m, 1H), 2.02-2.14 (m, 1H), 1.92-2.01 (m, 2H), 1.87 (br s, 4H).

$^1H$ NMR analysis shows that the salt formation number of crystal form C of the compound of formula (II) is 1.

**Example 5: Stability Test for Crystal Form A of Compound of Formula (II)**

<u>Procedure</u>

50 mg of crystal form A of the compound of formula (II) was measured and placed in a dry, clean glass bottle, spread into a thin layer, and left to stand under accelerated conditions (60 °C, 25/92.5% RH, 40 °C/75%RH and 60 °C/75%RH). The sample was completely exposed, and the bottle was covered with aluminum foil in which holes are pricked. Samples were taken at 5 days, 10 days, 1 month, 2 months and 3 months and analyzed. The results are shown in Table 6.

**Table 6. The results of the stability test for crystal form A of the compound of formula (II)**

| RRT | 0.23 | 0.31 | 0.91 | 0.98 | 0.99 | 1.08 | 1.40 | TRS (%) | Appearance | Crystal form | ee% |
|---|---|---|---|---|---|---|---|---|---|---|---|
| 0 days | / | 0.02 | / | / | / | / | / | / | White Solid | Crystal form A | 99.028 |
| 60 °C-5 days | 0.03 | 0.03 | / | / | / | / | / | 0.06 | White Solid | Crystal form A | 98.632 |
| 60 °C-10 days | 0.02 | 0.03 | / | / | / | 0.05 | / | 0.10 | White Solid | Crystal form A | 98.900 |
| 25/92.5% RH-5 days | / | 0.03 | / | / | / | / | / | 0.03 | White Solid | Crystal form A | 98.77 |
| 25/92.5% RH-10 days | / | 0.03 | / | / | / | / | 0.03 | 0.06 | White Solid | Crystal form A | 98.958 |
| 40°C/75% RH-10 days | 0.02 | 0.02 | / | / | / | / | / | 0.04 | White Solid | Crystal form A | 99.164 |
| 40 °C/75% RH-1 month | / | 0.02 | / | / | / | / | / | 0.02 | White Solid | Crystal form A | 99.380 |
| 40 °C/75% RH-2 months | / | / | 0.02 | / | / | / | / | 0.02 | White Solid | Crystal form A | 100 |
| 40 °C/75% RH-3 months | / | / | / | / | / | / | / | 0 | White Solid | Crystal form A | 100 |
| 60 °C/75% RH-10 days | 0.03 | / | | / | / | / | / | 0.03 | White Solid | Crystal form A | 98.872 |
| 60 °C/75% RH-1 month | 0.02 | / | / | 0.03 | 0.03 | / | | 0.08 | White Solid | Crystal form A | 100 |
| RRT: relative main peak retention time; TRS: total impurity content; RH: relative humidity; "/" indicates undetectable. | | | | | | | | | | | |

Conclusion: the impurity content of crystal form A of the compound of formula (II) substantially did not change under high temperature and high humidity conditions—the stability is good.

**Example 6: Hygroscopicity Test for Crystal Form A of Compound of Formula (II)**

Method:

A dry glass weighing bottle with a lid was placed in a dryer with saturated ammonium chloride solution placed in the lower part, and the weighing bottle was left to stand uncovered. A lid was put on the dryer, and the dryer was placed in a 25 °C box and left to stand overnight. After being left to stand overnight, the weighing bottle was taken out and precisely weighed, and the weight was denoted by $m_1$.

A proper amount of crystal form A of the compound of formula (II) was measured out and spread in the weighed measuring bottle (the sample was about 1 mm thick). The measuring bottled was then precisely weighed, and the weight was denoted by $m_2$.

The weighing bottle, together with its lid, was placed in a dryer with saturated ammonium chloride solution placed in the lower part and left to stand uncovered. A lid was put on the dryer, and the dryer was placed in a 25 °C box and left to stand for 24 h. After 24 h of sitting, the lid was put on the weighing bottle and the bottle was taken out and precisely weighed, the weight denoted by $m_3$.

The hygroscopic weight gain was calculated using the formula: percent weight gain = 100% $\times$ $(m_3 - m_2)/(m_2 - m_1)$.

Experimental results: crystal form A of the compound of formula (II) made a weight gain of 0.18% (mean). Conclusion: the mean hygroscopicity value of crystal form A of the compound of formula (II) is less than 0.2%, so crystal form A of the compound of formula (II) is non-hygroscopic or hardly hygroscopic.

**Example 7: Infrared Absorption Test for Crystal Form A of Compound of Formula (II)**

Instrument: Thermo Fisher Nicolet iS5

Method: attenuated total reflection (ATR)

Experimental results: the infrared absorption spectroscopic results of crystal form A of the compound of formula (II) are shown in Table 7. The infrared absorption spectrum of crystal form A of the compound of formula (II) is shown in FIG. 6.

**Table 7. The infrared absorption spectroscopic results of crystal form A of the compound of formula (II)**

| Absorption peak wavenumber ($cm^{-1}$) | Type of vibration | Group | Absorption peak intensity |
|---|---|---|---|
| 3225.69, 3033.88<br>2945.00, 2837.85 | NH, OH stretching | -NH, -OH | s |
| 2925.62, 3087.88 | C-H stretching | -CH, $-CH_2$, $-CH_3$ | s |
| 1680.20, 1662.75 | C-O stretching | C-O, -C=O | s |
| 1582.38 | C=C stretching | Benzene ring C=C | s |
| 1551.77 | C=N stretching | C=N | s |
| 1475.47, 1438.43 | $CH_2$ scissoring | $-CH_2$ | s |

Conclusion: the structure of the test compound contains such groups as -NH, -OH, benzene ring, $-CH_2$, $-CH_3$, C-O, -C=O and -C=N, agreeing with the structure of crystal form A of the compound of formula (II).

**Example 8: Ultraviolet Absorption Test for Crystal Form A of Compound of Formula (II)**

Instrument: Agilent G6860A ultraviolet-visible spectrophotometer
Experimental results: the ultraviolet results of crystal form A of the compound of formula (II) are shown in Table 8. The ultraviolet absorption spectrum of crystal form A of the compound of formula (II) is shown in FIG. 11.

**Table 8. Ultraviolet results**

| Solvent | Absorption wavelength (nm) | Absorbance (Abs) |
|---|---|---|
| $H_2O$ | 259.0 | 1.158 |
| | 214.4 | 1.356 |
| | 322.6 | 0.983 |

Analytical conclusion: the test compound has ultraviolet absorptions in water at $\lambda$ = 259.0 nm, $\lambda$ = 214.4 nm and $\lambda$ = 322.6 nm, which are B band and R band absorption peaks of an aromatic ring, indicating that the structure contains an aromatic ring, agreeing with the structure of crystal form A of the compound of formula (II).

**Example 9: AKT Enzymatic Activity Assay for Crystal Form A of Compound of Formula (II)**

CDC7 kinase plays an important role in the initiation of DNA replication and DNA damage response. When CDC7 is abnormally highly expressed, the abnormal activation of the CDC7-MCM2 signal pathway axis will cause

abnormal replication of DNA, and the activity of CHK2 and MK2, which are key checkpoint proteins for DNA damage repair, are inhibited, such that apoptosis is inhibited and proliferation is promoted, leading to tumorigenesis.

**Principle:**

The ADP-Glo™ kinase assay is a chemiluminescent kinase assay. The reaction of kinase with substrate requires ATP to provide energy, thereby converting ATP into ADP. The enzymatic activity is reflected by the amount of ADP produced in the reaction. In the assay, ADP is converted into ATP, and then ATP binds to Ultra-Glo™ Luciferase with light emitted; the intensity of light emission is directly proportional to the amount of ADP.

The ADP-GloTM assay was used to investigate the inhibitory effect of crystal form A of the test compound of formula (II) on the activity of CDC7/DBF4 kinase. The highest concentration of the test compound was 10 μM, and 5-fold serial dilution was performed to obtain 8 concentration points. The compound was re-tested on a different day, and each test was performed in duplicate.

**Reagents and consumables:**

| Name | Manufacturer |
|---|---|
| 384-well microplate | Greiner |
| 384-well compound plate | Costar |
| CDC7/DBF4 Active | Signal Chem |
| PDKtide | Signal Chem |
| Kinase assay buffer III | Signal Chem |
| DTT(0.1 M) | Signal Chem |
| ADP-Glo Kinase Assay | Promega |

**Main instrument:**

| Name | Manufacturer | Model |
|---|---|---|
| VICTOR Nivo | PerkinElmer | VICTOR Nivo 5F |

**Method:**

1. Solution and buffer preparation

1) Preparation of 10 mM DTT (freshly prepared at the time of use)
1 μL of 0.1 M DTT was measured out and 9 μL of deionized water was added.
2) 2× assay buffer (freshly prepared at the time of use)
168 μL of Kinase assay buffer III was measured out and 2.5-fold diluted with 248 μL of deionized water, and 4.2 μL of 10 mM DTT was added. The buffer was kept on ice before use.
3) 1× assay buffer (freshly prepared at the time of use)
250 μL of 2× assay buffer was measured out and diluted into 1× assay buffer by adding 250 μL of deionized water. The buffer was kept on ice before use.
4) ADP-Glo Kinase Assay

The kit contains an ADP-GloTM reagent, a kinase assay solution, a kinase assay solution matrix and a 10 mM ATP stock solution.

Preparation of the kinase assay solution: The kinase assay buffer and the kinase assay solution matrix were equilibrated to room temperature and then mixed. After complete dissolution, the solution was ready. The unused solution should be aliquoted and stored at -20 °C.

ADP-GloTM reagent: The reagent was equilibrated to room temperature at the time of the first use. The unused solution should be aliquoted and stored at -20 °C.

2. Preparation of compound working solution concentrations

1) 10 mM test compound was diluted to 1 mM with 100% DMSO: 5 μL of 10 mM test compound was added to 45 μL of 100% DMSO; the concentration of the compound dilution was 1 mM, and the dilution was put into row A of a 384-well compound plate as the highest concentration.

2) To row B through row H of the 384-well compound plate was added 20 μL of 100% DMSO.

3) From row A, 5 μL of compound was transferred to row B, and the mixture was well mixed. Then 5 μL of compound was transferred from row B to row C, and the mixture was well mixed. This procedure was repeated to row H to complete the serial dilution of the compound.

4) Preparation of compound working solutions: 0.5 μL/well of compound was transferred to a new 384-well compound plate, and 9.5 μL of 1× assay buffer was added. To a positive control well was added 0.5 μL of 100% DMSO followed by 9.5 μL of 1 × assay buffer.

5) The blank control well was a well where no CDC7/DBF4 enzyme was added, and the positive control was 1% DMSO.

3. Reaction steps

1) The CDC7/DBF4 enzyme stock solution, substrate PDKtide and ATP stock solution were thawed on ice and should be kept on ice during the experiment. The unused stock solutions should be aliquoted and stored to avoid repetition of freezing and thawing.

2) To a 384 microplate was added 1 μL/well of compound working solution. To a positive control well was added 1 μL/well of 1× assay buffer containing 5% DMSO. To a blank control well was added 1 μL/well of 1× assay buffer.

3) After the enzyme was completely dissolved, the enzyme stock solution was diluted with 1× assay buffer to 3.125 ng/μL-that is, 7 μL of the enzyme stock solution (100 ng/μL) was diluted with 217 μL of 1× assay buffer.

4) To the microplate was added 2 μL/well of enzyme solution. To a blank control well was added 2 μL/well of 1× assay buffer. By now the enzyme amount was 6.25 ng/well. Note: this step should be performed on ice.

5) 125 μL of substrate PDKtide stock solution was 2-fold diluted with a mixture of 122 μL of 2× assay buffer and 3.2 μL of ATP (2 mM). In the resulting mixture of substrate and ATP, the ATP concentration was 25 μM, and the PDKtide concentration was 0.5 mg/mL. The mixed solution of substrate and ATP was kept on ice before use.

6) To the microplate was added 2 μL/well of the mixed solution of substrate and ATP. By now the substrate concentration was 0.2 mg/mL, the ATP concentration was 10 μM, and the DMSO concentration was 1%.

7) The microplate was sealed with a membrane and incubated at 25 °C for 60 min.

8) The ADP-GloTM reagent and kinase assay solution should be equilibrated to room temperature before use.

9) After the incubation, 5 μL/well of ADP-GloTM reagent was added to the microplate, and the microplate was sealed with a membrane and then incubated at 25 ° C for 40 min.

10) After the incubation, 10 μL/well of kinase assay solution was added to the microplate, and the microplate was sealed with a membrane and then incubated at 25 ° C for 30 min.

11) After the incubation, luminescence was measured on Nivo, and luminescence readings (RLU) were taken.

12) The enzymatic activity rate was calculated:

$$\% \text{ enzymatic activity} = [(\text{RLU(compound)} - \text{RLU(blank)}) / (\text{RLU(positive)} - \text{RLU(blank)})] \times 100\%$$

**Experimental results**

The percent enzymatic activity was calculated from the original readings according to the calculation formula. A graph was then plotted using Prism and the $IC_{50}$ of the compound was calculated. The results are shown in the table below.

| Compound | $IC_{50}$ (nM) | | |
|---|---|---|---|
| | First assay | Second assay | Mean |
| | | | |
| Crystal form A of the compound of formula (II) | 3.49 | 2.35 | 2.92±0.81 |

**Conclusion:** crystal form A of the compound of formula (II) has a relatively strong inhibitory effect on enzyme CDC7/DBF4.

**Example 10: Cell Viability Assay for Crystal Form A of Compound of Formula (II)**

In the assay, the inhibitory effect of crystal form A of the compound of formula (II) on the proliferation of colorectal cell COLO205 that highly expresses Cdc7 was investigated.

**Reagents and consumables:**

1) Compound

Crystal form A of the compound of formula (II).

2) Main instrument

| **Name** | **Manufacturer** | **Model** |
|---|---|---|
| Envision | PerkinFlmer | EXT |

3) Cell

| Cell type | Cell name | Cell growth pattern | Cell culture medium | Cell source |
|---|---|---|---|---|
| Human colorectal cancer cell | Colo-205 | Adhesion | RPMI 1640 + 10% FBS + 1% bispecific antibody | Procell Biotech |

4) Main reagents and consumables

1640 medium, Biological Industries; bispecific antibody, Procell; 0.25% pancreatin, Base Media; fetal bovine serum, Biosera; DMSO, Sinopharm; 96-well cell culture plate, Corning; 96-well compound plate, Shanghai Jingrao Biotech Ltd.; CellTiter-Glo® Luminescent Cell Viability Assay, Promega; cell culture dish, Nest.

**Principle:**

CellTiter-Glo® Luminescent Cell Viability Assay is a homogeneous rapid assay for the viability of living cells in cultures by ATP quantification. ATP is an indicator of the metabolism of living cells. Cell lysis and the luminescence signals generated are directly proportional to the amount of ATP that exists, and the amount of ATP is directly proportional to the number of living cells in cultures.

**Method:**

1) Cell passage

Cells having achieved 80%-90% cell fusion were digested with pancreatin. After the digestion was stopped with 1 mL of culture medium, the cells were transferred to a new culture dish according to a proper proportion, and 10 mL of fresh cell culture medium was added to the culture dish. Then the cell culture dish was placed in a 37 °C, 5% $CO_2$ incubator to continue the culture.

2) Preparation of compound working solution concentrations

The compound was diluted to 2 mM with 100% DMSO-that is, 5 μL of 10 mM compound stock solution was transferred to column 1 of a 96-well compound plate, 20 μL of 100% DMSO was then added, and the mixture was well mixed. To column 2 through column 9 of the 96-well compound plate was added 20 μL of 100% DMSO. From column 1, 10 μL of compound was transferred to column 2, and the mixture was well mixed. Then 10 μL of compound was transferred from column 2 to column 3, and the mixture was well mixed. This procedure was repeated to column 9 to complete the serial dilution of the compound. Preparation of compound working solutions: From the compound plate, 2 μL/well of compound was transferred to a new 96-well compound plate, and 78 μL/well of cell culture medium was added. To a negative control well was added 2 μL of 100% DMSO followed by 78 μL of cell culture medium. The compound concentration was the final concentration. The blank control well was a culture medium well containing no cells. The negative control was 0.5% DMSO.

3) Cell inoculation and drug treatment

COLO205 cells having achieved 80%-90% cell fusion were digested with pancreatin and counted. After counting, the cell suspension was diluted with cell culture medium to a desired density. The cell density is shown in the table below. To each well of a 96-well cell culture plate was inoculated 80 μL of cell suspension. The plate was incubated in a 37 °C, 5% $CO_2$ incubator overnight. On the day of the experiment, 20 μL of compound working solution was added to each well of the cell plate, and the plate was incubated in a 37 °C, 5% $CO_2$ incubator for another period of time. The specific cell plating density and the number of days of incubation are shown in the table below.

The plating density of COLO205 cells and the length of the co-incubation of the cells and the compound

| Cell name | Plating density (cells/well) | Length of incubation with compound (days) |
|---|---|---|
| COLO205 | 3000 | 3 |

After the incubation, 100 μL of CTG assay reagent was added to each well of the cell plate, and the plate was incubated at 25 °C for 10 min. After the incubation, luminescence signals were measured on Envision.

**Experimental results:**

The signal value of the background blank control well was subtracted from the measured signal value of each group for standardization. The standardized data were used to calculate the cell viability after compound treatment according to the following formula: %inhibition = [1 - ($RFU_{compound}$ - $RFU_{blank}$ control) / ($RFU_{positive\ control}$ - $RFU_{blank\ control}$)]

× 100%. Blank control: a treatment involving no cells but culture medium only. Positive control: cells treated with 0.5% DMSO; a graph was then plotted using Prism and the $IC_{50}$ value of the compound was calculated.

The percent inhibition was calculated from the original readings according to the calculation formula. A graph was then plotted using Prism and the $IC_{50}$ of the compound was calculated.

The inhibitory effect $IC_{50}$ of crystal form A of the compound of formula (II) on the proliferation of COLO205 cells is shown in the table below.

| Compound name | COLO205(nM) | | |
|---|---|---|---|
| | First assay | Second assay | Mean |
| Crystal form A of the compound of formula (II) | 14.34 | 14.72 | 14.53±0.27 |

Conclusion: crystal form A of the compound of formula (II) has a relatively strong inhibitory effect on COLO205 cells.

**Example 11: Confirmation of Steric Configuration of Compound of Formula (I)**

The steric configuration of the compound of formula (I) was confirmed by single-crystal X-ray diffraction. Preparation of single crystal of compound of formula (I):

A 20-mg sample of the compound of formula (I) was measured out and dissolved in 1 mL of methanol/methyl tert-butyl ether (1:1) at room temperature, and the sample solution was placed in a 4 mL semi-sealed sample bottle to allow the solvents to be slowly volatilized at room temperature. A colorless columnar crystal was obtained on day three and its structure was analyzed by single-crystal X-ray diffraction.

Experimental results: the ellipsoid plot of the three-dimensional structure of the compound of formula (I) is shown in FIG. 12 and indicates an S configuration. The structural data and parameters of the crystal of the compound of formula (I) are shown in Tables 9, 10, 11 and 12.

**Table 9. The structural data and measurement parameters of the crystal of the compound of formula (I)**

| Parameter | Crystal of compound of formula (I) |
|---|---|
| Experiment | Single-crystal X-ray diffraction |
| Molecular weight | 367.47 |
| Temperature | 100.2(7)K |
| Wavelength | 1.54184Å |
| Crystal system, space group | Monoclinic, $P2_1$ |
| Dimensions of unit cell | a = 10.79027(8) Å<br>b = 7.07640(4) Å<br>c = 11.65192(13) Å<br>$\alpha$ = 90°<br>$\beta$ = 107.4021(10)°<br>$\gamma$ = 90° |
| Volume | 848.974(13) $Å^3$ |
| Z, calculated density | 2, 1.437$Mg/m^3$ |
| Absorption coefficient | 1.852$mm^{-1}$ |
| F(000) | 388.0 |
| Dimensions of crystal | 0.40 × 0.10 × 0.10 mm |
| Angle range for data collection | 7.952 to 133.152°deg. |
| Limiting index | -12 h 12, -8 k 8, -13 1 11 |
| Reflection collection/uniqueness | 29867 / 2973 [R(int) = 0.0731] |

**Table 10. The atomic coordinates (×10$^4$) and equivalent isotropic displacement parameters (Å$^2$ × 10$^3$) of the crystal of the compound of formula (I)**

| | x | y | z | U(eq) |
|---|---|---|---|---|
| S(1) | -9198.8(5) | -2477.2(8) | -9046.0(5) | 13.62(17) |
| N(3) | -9633.4(18) | -2566(4) | -12504.5(18) | 14.5(4) |
| N(4) | -7382.9(19) | -2306(3) | -11549.5(19) | 15.4(5) |
| N(5) | -6799.4(19) | -2265(4) | -13637.7(19) | 15.7(5) |
| C(11) | -9769(2) | -2594(4) | -11361(2) | 13.3(5) |
| O(1) | -6414.0(16) | -2223(3) | -9505.8(16) | 18.2(4) |
| C(4) | -11810(2) | -2603(4) | -9284(2) | 13.5(5) |
| C(12) | -8448(2) | -2389(4) | -12538(2) | 14.8(5) |
| C(1) | -7416(2) | -2336(4) | -10369(2) | 14.4(5) |
| N(2) | -13650(2) | -2593(4) | -8855(2) | 16.4(5) |
| C(6) | -13147(2) | -2436(4) | -9779(2) | 14.3(5) |
| C(10) | -10992(2) | -2651(4) | -11116(2) | 13.4(5) |
| C(5) | -11626(2) | -2829(4) | -8038(2) | 16.0(6) |
| C(3) | -10828(2) | -2584(4) | -9903(2) | 14.0(5) |
| C(2) | -8718(2) | -2499(4) | -10321(2) | 14.2(5) |
| N(1) | -12736(2) | -2839(3) | -7767(2) | 18.8(5) |
| C(9) | -12276(2) | -2805(4) | -12083(2) | 16.6(6) |
| C(16) | -7612(3) | -1984(4) | -15874(3) | 21.4(6) |
| C(17) | -6612(3) | -1287(4) | -14692(3) | 21.3(6) |
| C(13) | -8196(2) | -2109(4) | -13739(2) | 15.3(6) |
| C(8) | -13330(3) | -1465(4) | -11937(3) | 16.9(6) |
| C(19) | -6441(3) | -4276(4) | -13691(3) | 18.5(6) |
| C(7) | -14010(2) | -2138(4) | -11034(2) | 17.4(6) |
| C(15) | -8218(3) | -3823(4) | -15597(3) | 18.7(6) |
| C(18) | -7124(3) | -5161(4) | -14938(3) | 22.3(6) |
| C(14) | -9035(3) | -3386(4) | -14752(2) | 17.1(6) |

**Table 11. The bond lengths (Å) and bond angles [deg] of the crystal of the compound of formula (I)**

| Bond length | Bond angle | Bond length | Bond angle |
|---|---|---|---|
| S(1) C(3) | 1.745(2) | C(1) C(2) | 1.428(3) |
| S(1) C(2) | 1.714(2) | N(2) C(6) | 1.348(3) |
| N(3) C(11) | 1.383(3) | N(2) N(1) | 1.365(3) |
| N(3)C(12) | 1.297(3) | C(6)C(7) | 1.495(4) |
| N(4)C(12) | 1.364(3) | C(10)C(3) | 1.372(4) |
| N(4)C(1) | 1.387(3) | C(10)C(9) | 1.506(3) |
| N(5)C(17) | 1.476(4) | C(5)N(1) | 1.327(4) |
| N(5)C(13) | 1.480(3) | C(9)C(8) | 1.529(4) |
| N(5)C(19) | 1.481(4) | C(16)C(17) | 1.554(4) |
| C(11)C(10) | 1.432(3) | C(16)C(15) | 1.534(4) |
| C(11)C(2) | 1.391(3) | C(13)C(14) | 1.546(4) |
| O(1)C(1) | 1.240(3) | C(8)C(7) | 1.527(4) |
| C(4)C(6) | 1.389(3) | C(19)C(18) | 1.551(4) |
| C(4)C(5) | 1.414(4) | C(15)C(18) | 1.528(4) |
| C(4)C(3) | 1.449(3) | C(15)C(14) | 1.537(4) |
| C(12)C(13) | 1.517(4) | | |

**Table 12. The torsion angles [deg] of the crystal of the compound of formula (I)**

| | | | |
|---|---|---|---|
| N(3) C(11) C(10) C(3) | -177.3(3) | C(10) C(9) C(8) C(7) | -80.2(3) |
| N(3) C(11) C(10) C(9) | 3.8(5) | C(5) C(4) C(6) N(2) | -0.9(3) |
| N(3) C(11) C(2) S(1) | 177.8(2) | C(5) C(4) C(6) C(7) | 178.9(3) |
| N(3) C(11) C(2) C(1) | 1.0(4) | C(5) C(4) C(3) S(1) | -10.3(4) |
| N(3) C(12) C(13) N(5) | -170.1(3) | C(5) C(4) C(3) C(10) | 169.2(3) |
| N(3) C(12) C(13) C(14) | -42.4(4) | C(3) S(1) C(2) C(11) | -0.3(2) |
| N(4) C(12) C(13) N(5) | 14.2(3) | C(3) S(1) C(2) C(1) | 176.4(3) |
| N(4) C(12) C(13) C(14) | 141.8(3) | C(3) C(4) C(6) N(2) | 178.2(3) |
| N(4) C(1) C(2) S(1) | -176.8(2) | C(3) C(4) C(6) C(7) | -2.0(6) |
| N(4) C(1) C(2) C(11) | -0.4(4) | C(3) C(4) C(5) N(1) | -178.0(3) |
| N(5) C(13) C(14) C(15) | -20.2(3) | C(3) C(10) C(9) C(8) | 46.5(4) |
| N(5) C(19) C(18) C(15) | -14.7(3) | C(2) S(1) C(3) C(4) | -179.8(2) |
| C(11) N(3) C(12) N(4) | 2.5(4) | C(2) S(1) C(3) C(10) | 0.6(2) |
| C(11) N(3) C(12) C(13) | -172.8(2) | C(2) C(11) C(10) C(3) | 0.5(4) |
| C(11) C(10) C(3) S(1) | -0.7(3) | C(2) C(11) C(10) C(9) | -178.4(2) |
| C(10) C(11) C(3) C(4) | 179.8(3) | N(1) N(2) C(6) C(4) | 0.5(3) |
| C(11) C(10) C(9) C(8) | -134.8(3) | N(1) N(2) C(6) C(7) | -179.3(2) |
| O(1) C(1) C(2) S(1) | 2.9(5) | C(9) C(10) C(3) S(1) | 178.2(2) |
| O(1) C(1) C(2) C(11) | 179.2(3) | C(9) C(10) C(3) C(4) | -1.4(5) |
| C(4) C(6) C(7) C(8) | -15.4(5) | C(9) C(8) C(7) C(6) | 59.2(3) |
| C(4) C(5) N(1) N(2) | -0.7(3) | C(16) C(15) C(18) C(19) | 67.0(3) |
| C(12) N(3) C(11) C(10) | 175.7(3) | C(16) C(15) C(14) C(13) | -46.9(3) |
| C(12) N(3) C(11) C(2) | -1.9(4) | C(17) N(5) C(13) C(12) | -159.0(2) |
| C(12) N(4) C(1) O(1) | -178.7(3) | C(17) N(5) C(13) C(14) | 72.2(3) |
| C(12) N(4) C(1) C(2) | 1.0(4) | C(17) N(5) C(19) C(18) | -50.5(3) |
| C(12) C(13) C(14) C(15) | -148.1(2) | C(17) C(16) C(15) C(18) | -50.5(3) |
| C(1) N(4) C(12) N(3) | -2.2(5) | C(17) C(16) C(15) C(14) | 66.9(3) |
| C(1) N(4) C(12) C(13) | 173.2(2) | C(13) N(5) C(17) C(16) | -50.4(3) |
| N(2) C(6) C(7) C(8) | 164.4(3) | C(13) N(5) C(19) C(18) | 65.8(3) |
| C(6) C(4) C(5) N(1) | 1.0(3) | C(19) N(5) C(17) C(16) | 67.9(3) |
| C(6) C(4) C(3) S(1) | 170.9(3) | C(19) N(5) C(13) C(12) | 83.8(3) |
| C(6) C(4) C(3) C(10) | -9.6(5) | C(19) N(5) C(13) C(14) | -45.0(3) |
| C(6) N(2) N(1) C(5) | 0.1(3) | C(15) C(16) C(17) N(5) | -15.2(3) |
| C(10) C(11) C(2) S(1) | 0.0(3) | C(18) C(15) C(14) C(13) | 70.9(3) |
| C(10) C(11) C(2) C(1) | -176.9(3) | C(14) C(15) C(18) C(19) | -51.0(3) |

## Claims

1. A compound of formula (II) or a crystal form thereof:

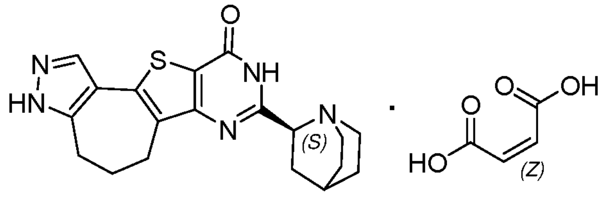

(II)

2. The crystal form of the compound of formula (II) according to claim 1, wherein an X-ray powder diffraction pattern

of the crystal form comprises characteristic peaks at 2θ values of 11.46 ± 0.20°, 24.03 ± 0.20° and 25.16 ± 0.20°.

**3.** The crystal form of the compound of formula (II) according to claim 2, wherein the X-ray powder diffraction pattern of the crystal form comprises characteristic peaks at 2Θ values of 11.46 ± 0.20°, 12.06 ± 0.20°, 16.96 ± 0.20°, 17.60 ± 0.20°, 18.48 ± 0.20°, 19.55 ± 0.20°, 24.03 ± 0.20° and 25.16 ± 0.20°; or the X-ray powder diffraction pattern of the crystal form comprises characteristic peaks at 2Θ values of 6.46° ± 0.20°, 9.36° ± 0.20°, 11.46° ± 0.20°, 12.06° ± 0.20°, 12.39° ± 0.20°, 12.89° ± 0.20°, 13.37° ± 0.20°, 13.87° ± 0.20°, 14.09° ± 0.20°, 16.10° ± 0.20°, 16.96° ± 0.20°, 17.19° ± 0.20°, 17.60° ± 0.20°, 18.48° ± 0.20°, 18.75° ± 0.20°, 19.37° ± 0.20°, 19.55° ± 0.20°, 21.21° ± 0.20°, 21.65° ± 0.20°, 22.36° ± 0.20°, 23.06° ± 0.20°, 23.42° ± 0.20°, 23.74° ± 0.20°, 24.03° ± 0.20°, 25.16° ± 0.20°, 25.47° ± 0.20°, 26.59° ± 0.20°, 27.32° ± 0.20°, 28.11° ± 0.20°, 28.77° ± 0.20°, 29.73° ± 0.20°, 31.81° ± 0.20°, 33.09° ± 0.20°, 33.80° ± 0.20°, 34.19° ± 0.20°, 35.49° ± 0.20°, 35.99° ± 0.20°, 37.66° ± 0.20°, 38.14° ± 0.20°, 38.77° ± 0.20° and 39.38° ± 0.20°; or the crystal form has the following X-ray powder diffraction pattern data:

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 1 | 6.46 | 13.69 | 4.02 | 22 | 23.42 | 3.80 | 6.95 |
| 2 | 9.36 | 9.45 | 11.84 | 23 | 23.74 | 3.75 | 10.34 |
| 3 | 11.46 | 7.72 | 50.71 | 24 | 24.03 | 3.70 | 100.00 |
| 4 | 12.06 | 7.34 | 22.16 | 25 | 25.16 | 3.54 | 29.42 |
| 5 | 12.39 | 7.14 | 4.33 | 26 | 25.47 | 3.50 | 5.01 |
| 6 | 12.89 | 6.87 | 3.05 | 27 | 26.59 | 3.35 | 1.75 |
| 7 | 13.37 | 6.62 | 7.19 | 28 | 27.32 | 3.26 | 6.12 |
| 8 | 13.87 | 6.39 | 6.58 | 29 | 28.11 | 3.17 | 5.99 |
| 9 | 14.09 | 6.29 | 6.09 | 30 | 28.77 | 3.10 | 2.43 |
| 10 | 16.10 | 5.51 | 5.75 | 31 | 29.73 | 3.01 | 2.79 |
| 11 | 16.96 | 5.23 | 15.15 | 32 | 31.81 | 2.81 | 2.39 |
| 12 | 17.19 | 5.16 | 14.98 | 33 | 33.09 | 2.71 | 1.82 |
| 13 | 17.60 | 5.04 | 15.82 | 34 | 33.80 | 2.65 | 1.63 |
| 14 | 18.48 | 4.80 | 18.58 | 35 | 34.19 | 2.62 | 2.12 |
| 15 | 18.75 | 4.73 | 5.85 | 36 | 35.49 | 2.53 | 1.08 |
| 16 | 19.37 | 4.58 | 11.94 | 37 | 35.99 | 2.50 | 1.06 |
| 17 | 19.55 | 4.54 | 13.94 | 38 | 37.66 | 2.39 | 1.81 |
| 18 | 21.21 | 4.19 | 9.40 | 39 | 38.14 | 2.36 | 0.74 |
| 19 | 21.65 | 4.11 | 6.66 | 40 | 38.77 | 2.32 | 1.55 |
| 20 | 22.36 | 3.98 | 6.35 | 41 | 39.38 | 2.29 | 1.41 |
| 21 | 23.06 | 3.86 | 6.57 | | | | |

typically, the X-ray powder diffraction pattern is as shown in FIG. 1.

**4.** The crystal form of the compound of formula (II) according to any one of claims 2-3, wherein a DSC curve of the crystal form has an endothermic peak starting point at 230.7 °C ± 3 °C; typically, a DSC profile of the crystal form is as shown in FIG. 4.

**5.** The crystal form of the compound of formula (II) according to claim 1, wherein an X-ray powder diffraction pattern of the crystal form comprises characteristic peaks at 2Θ values of 5.86 ± 0.20°, 17.64 ± 0.20° and 24.89 ± 0.20°.

6. The crystal form of the compound of formula (II) according to claim 5, wherein the X-ray powder diffraction pattern of the crystal form comprises characteristic peaks at 2Θ values of 5.86 ± 0.20°, 10.17 ± 0.20°, 11.73 ± 0.20°, 15.83 ± 0.20°, 17.64 ± 0.20°, 23.59 ± 0.20°, 24.89 ± 0.20° and 25.80 ± 0.20°; or the X-ray powder diffraction pattern of the crystal form comprises characteristic peaks at 2Θ values of 5.86° ± 0.20°, 10.17° ± 0.20°, 11.73° ± 0.20°, 12.57° ± 0.20°, 14.15° ± 0.20°, 14.40° ± 0.20°, 15.23° ± 0.20°, 15.83° ± 0.20°, 16.26° ± 0.20°, 16.71° ± 0.20°, 17.64° ± 0.20°, 18.04° ± 0.20°, 18.73° ± 0.20°, 19.99° ± 0.20°, 20.57° ± 0.20°, 21.08° ± 0.20°, 23.59° ± 0.20°, 24.36° ± 0.20°, 24.89° ± 0.20°, 25.41° ± 0.20°, 25.80° ± 0.20°, 27.20° ± 0.20°, 27.90° ± 0.20°, 28.90° ± 0.20°, 29.39° ± 0.20°, 29.70° ± 0.20°, 30.52° ± 0.20°, 30.81° ± 0.20°, 31.99° ± 0.20°, 34.29° ± 0.20°, 35.83° ± 0.20°, 39.64° ± 0.20°, 28.90° ± 0.20°, 29.39° ± 0.20°, 29.70° ± 0.20°, 30.52° ± 0.20°, 30.81° ± 0.20°, 31.99° ± 0.20°, 34.29° ± 0.20°, 35.83° ± 0.20° and 39.64° ± 0.20°; or the crystal form has the following X-ray powder diffraction pattern data:

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 1 | 5.86 | 15.09 | 91.11 | 22 | 27.20 | 3.28 | 5.62 |
| 2 | 10.17 | 8.70 | 45.62 | 23 | 27.90 | 3.20 | 5.66 |
| 3 | 11.73 | 7.55 | 22.38 | 24 | 28.90 | 3.09 | 7.73 |
| 4 | 12.57 | 7.04 | 4.09 | 25 | 29.39 | 3.04 | 4.33 |
| 5 | 14.15 | 6.26 | 13.40 | 26 | 29.70 | 3.01 | 7.45 |
| 6 | 14.40 | 6.15 | 16.28 | 27 | 30.52 | 2.93 | 4.19 |
| 7 | 15.23 | 5.82 | 4.48 | 28 | 30.81 | 2.90 | 4.70 |
| 8 | 15.83 | 5.60 | 16.95 | 29 | 31.99 | 2.80 | 2.25 |
| 9 | 16.26 | 5.45 | 5.96 | 30 | 34.29 | 2.61 | 3.87 |
| 10 | 16.71 | 5.30 | 9.51 | 31 | 35.83 | 2.51 | 4.54 |
| 11 | 17.64 | 5.03 | 100.00 | 32 | 39.64 | 2.27 | 4.83 |
| 12 | 18.04 | 4.92 | 10.69 | 33 | 28.90 | 3.09 | 7.73 |
| 13 | 18.73 | 4.74 | 8.44 | 34 | 29.39 | 3.04 | 4.33 |
| 14 | 19.99 | 4.44 | 5.96 | 35 | 29.70 | 3.01 | 7.45 |
| 15 | 20.57 | 4.32 | 2.74 | 36 | 30.52 | 2.93 | 4.19 |
| 16 | 21.08 | 4.21 | 4.51 | 37 | 30.81 | 2.90 | 4.70 |
| 17 | 23.59 | 3.77 | 45.03 | 38 | 31.99 | 2.80 | 2.25 |
| 18 | 24.36 | 3.65 | 4.48 | 39 | 34.29 | 2.61 | 3.87 |
| 19 | 24.89 | 3.58 | 55.05 | 40 | 35.83 | 2.51 | 4.54 |
| 20 | 25.41 | 3.51 | 26.82 | 41 | 39.64 | 2.27 | 4.83 |
| 21 | 25.80 | 3.45 | 22.73 | | | | |

typically, the X-ray powder diffraction pattern is as shown in FIG. 2.

7. The crystal form of the compound of formula (II) according to any one of claims 5-6, wherein a DSC curve of the crystal form has endothermic peak starting points at 65.1 °C ± 3 °C, 113.7 °C ± 3 °C, 208.8 °C ± 3 °C and 221.1 °C ± 3 °C; typically, a DSC profile of the crystal form is as shown in FIG. 7.

8. The crystal form of the compound of formula (II) according to claim 1, wherein an X-ray powder diffraction pattern of the crystal form comprises characteristic peaks at 2Θ values of 7.40 ± 0.20°, 11.21 ± 0.20° and 22.18 ± 0.20°.

9. The crystal form of the compound of formula (II) according to claim 8, wherein the X-ray powder diffraction pattern of the crystal form comprises characteristic peaks at 2Θ values of 7.40 ± 0.20°, 11.21 ± 0.20°, 13.95 ± 0.20°, 15.01

± 0.20°, 15.72 ± 0.20°, 20.61 ± 0.20°, 22.18 ± 0.20° and 23.82 ± 0.20°; or the X-ray powder diffraction pattern of the crystal form comprises characteristic peaks at 2Θ values of 7.40° ± 0.20°, 10.50° ± 0.20°, 11.21° ± 0.20°, 11.81° ± 0.20°, 13.05° ± 0.20°, 13.95° ± 0.20°, 15.01° ± 0.20°, 15.72°, 16.28°, 17.64°, 18.35°, 18.73°, 19.53°, 20.14°, 20.61°, 22.18°, 22.51°, 23.82°, 24.37°, 25.49°, 26.36° ± 0.20°, 27.19° ± 0.20°, 28.93° ± 0.20°, 30.70° ± 0.20°, 31.60° ± 0.20°, 32.50° ± 0.20° and 34.33° ± 0.20°; or the crystal form has the following X-ray powder diffraction pattern data:

| No. | 2θ angle (±0.2°) | Interplanar spacing (Å) | Relative intensity (%) | No. | 2θ angle (±0.2°) | Interplanar spacing (Å) | Relative intensity (%) |
|---|---|---|---|---|---|---|---|
| 1 | 7.40 | 11.94 | 57.67 | 15 | 20.61 | 4.31 | 29.68 |
| 2 | 10.50 | 8.43 | 9.35 | 16 | 22.18 | 4.01 | 80.86 |
| 3 | 11.21 | 7.90 | 100.00 | 17 | 22.51 | 3.95 | 20.92 |
| 4 | 11.81 | 7.49 | 8.63 | 18 | 23.82 | 3.74 | 39.44 |
| 5 | 13.05 | 6.79 | 3.42 | 19 | 24.37 | 3.65 | 4.57 |
| 6 | 13.95 | 6.35 | 46.42 | 20 | 25.49 | 3.49 | 17.03 |
| 7 | 15.01 | 5.90 | 31.30 | 21 | 26.36 | 3.38 | 3.79 |
| 8 | 15.72 | 5.64 | 38.00 | 22 | 27.19 | 3.28 | 17.79 |
| 9 | 16.28 | 5.45 | 13.70 | 23 | 28.93 | 3.09 | 11.30 |
| 10 | 17.64 | 5.03 | 19.39 | 24 | 30.70 | 2.91 | 3.90 |
| 11 | 18.35 | 4.84 | 12.39 | 25 | 31.60 | 2.83 | 2.44 |
| 12 | 18.73 | 4.74 | 18.09 | 26 | 32.50 | 2.75 | 3.43 |
| 13 | 19.53 | 4.55 | 7.13 | 27 | 34.33 | 2.61 | 3.67 |
| 14 | 20.14 | 4.41 | 2.40 | | | | |

typically, the X-ray powder diffraction pattern is as shown in FIG. 3.

**10.** The crystal form of the compound of formula (II) according to any one of claims 8-9, wherein a DSC curve of the crystal form has an endothermic peak starting point at 219.9 °C ± 3 °C; typically, a DSC profile of the crystal form is as shown in FIG. 9.

**11.** A method for preparing the crystal form of the compound of formula (II) according to any one of claims 1-10, wherein the method comprises the following steps:

(1) mixing a compound of formula (I) as shown below with methanol;
(2) adding maleic acid to the mixture of step (1);
(3) performing filtration and drying to obtain the crystal form according to claim 2 or the crystal form according to claim 8; and
(4) mixing the crystal form according to claim 2 with acetonitrile and water, and after the solid is dissolved, volatilizing the solvents completely to obtain the crystal form according to claim 5,

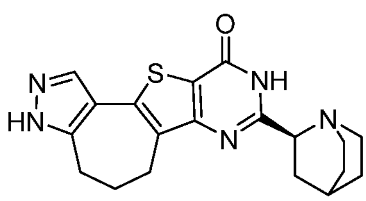

(I)

.

**12.** A crystalline composition comprising the crystal form of the compound of formula (II) according to any one of claims

1-10, wherein the crystal form of the compound of formula (II) according to any one of claims 1-10 makes up 50% or more, preferably 75% or more, more preferably 90% or more, and most preferably 95% or more by weight of the crystalline composition.

**13.** A pharmaceutical composition comprising the compound of formula (II) or the crystal form thereof according to any one of claims 1-10 or the crystalline composition according to claim 12, and optionally a pharmaceutically acceptable excipient.

**14.** Use of the compound of formula (II) or the crystal form thereof according to any one of claims 1-10, the crystalline composition according to claim 12 or the pharmaceutical composition according to claim 13 in preventing or treating a Cdc7 kinase-mediated disease, or for preparing a medicament for preventing or treating a Cdc7 kinase-mediated disease, wherein preferably, the Cdc7 kinase-mediated disease is a tumor; more preferably, the tumor is colorectal cancer or pancreatic cancer.

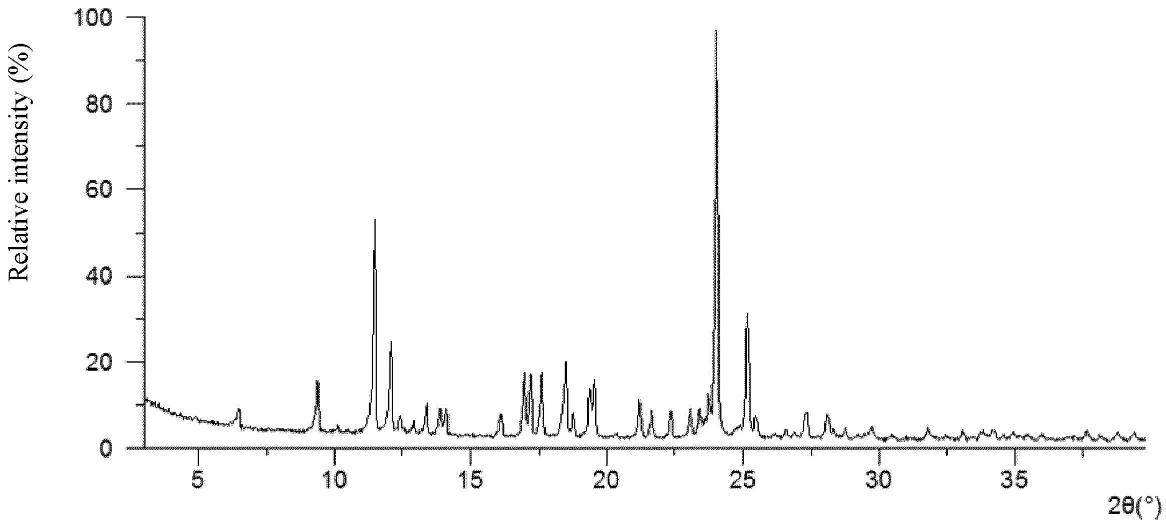


FIG. 1

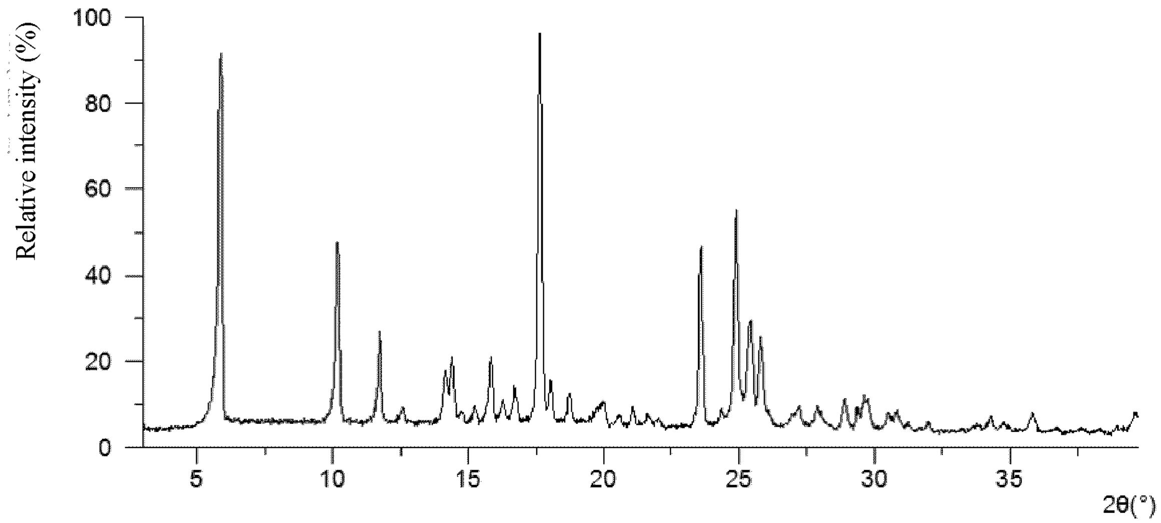


FIG. 2

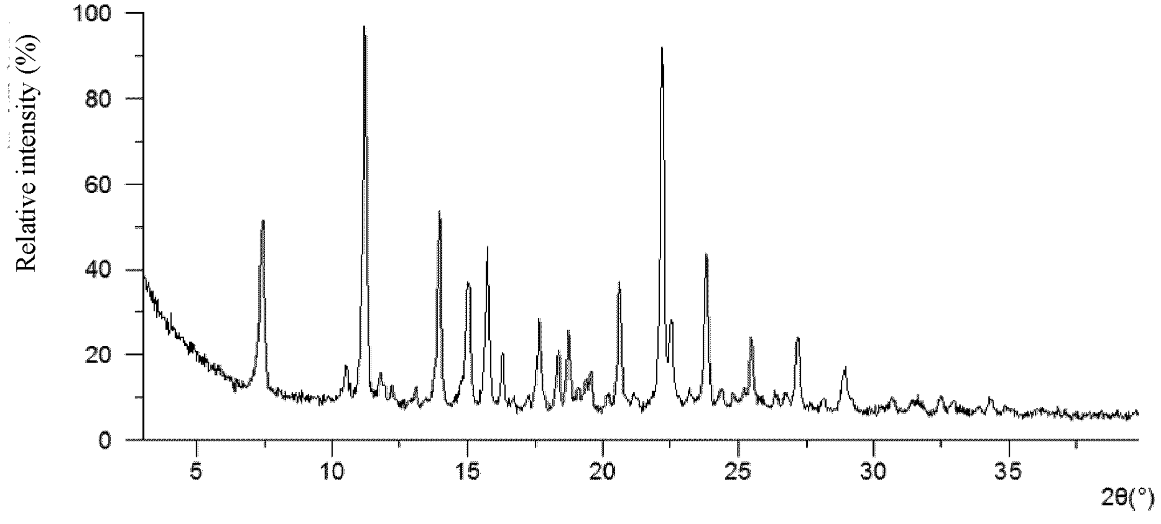


FIG. 3

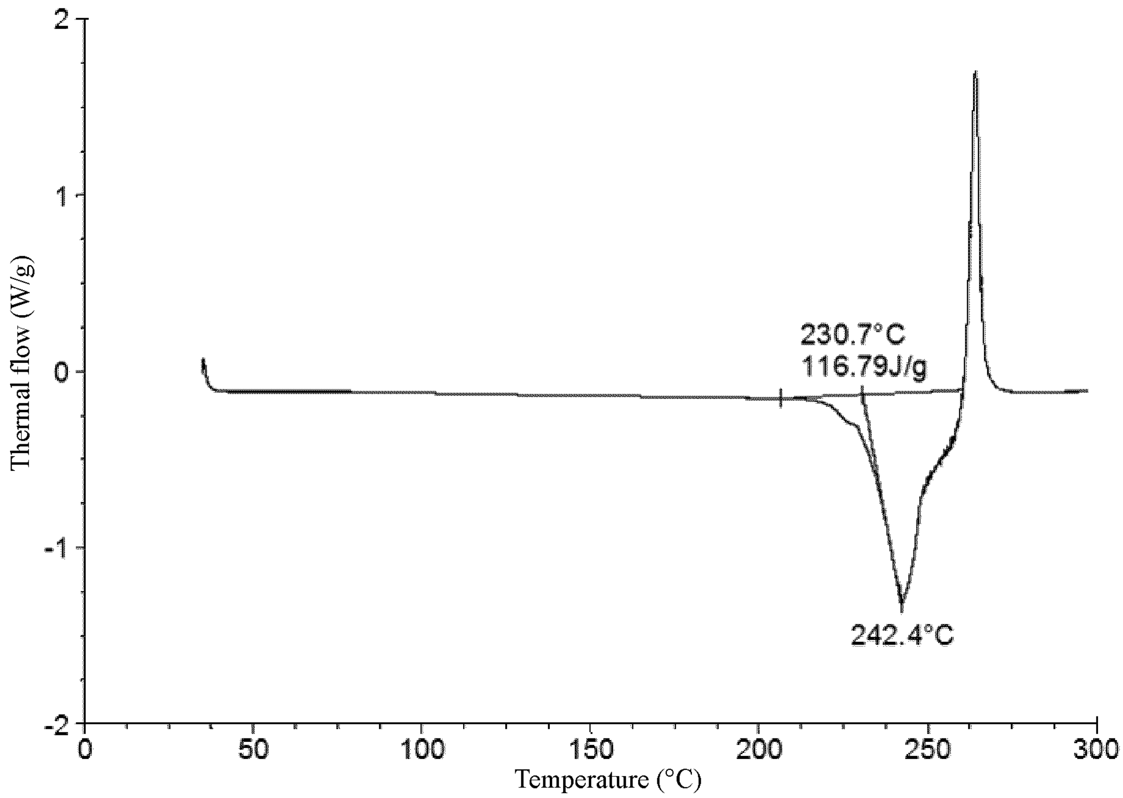
2
1
0
-1
-2
Thermal flow (W/g)
230.7°C
116.79J/g
242.4°C
0
50
100
150
200
250
300
Temperature (°C)

FIG. 4

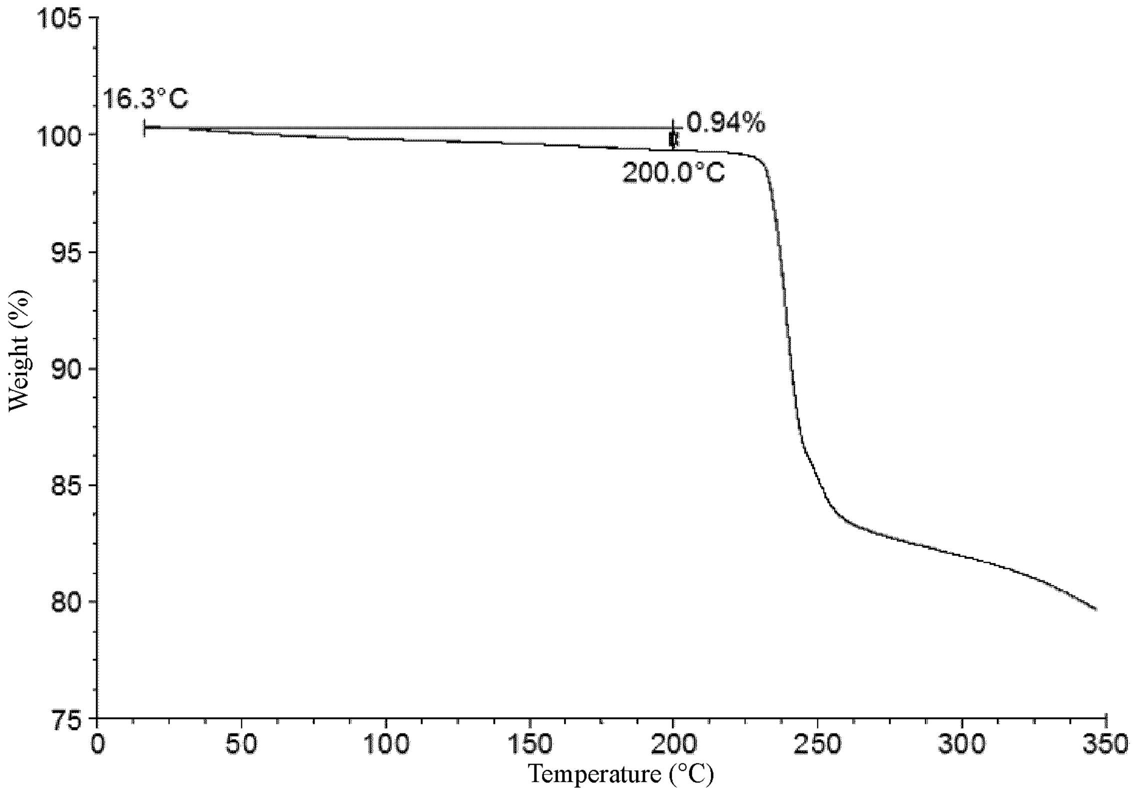
105
100
95
90
85
80
75
Weight (%)
16.3°C
0.94%
200.0°C
0
50
100
150
200
250
300
350
Temperature (°C)

FIG. 5

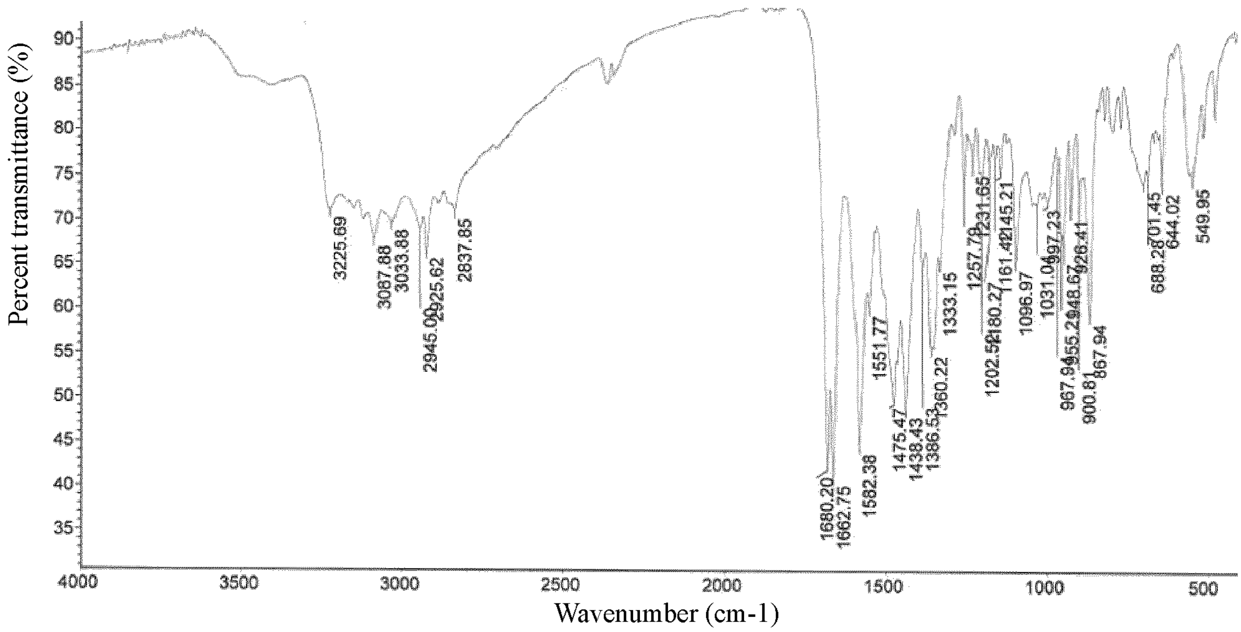
Percent transmittance (%)
Wavenumber (cm-1)
4000
3500
3000
2500
2000
1500
1000
500
3225.69
3087.88
3033.88
2837.85
1680.20
1662.75
1582.38
1551.77
1475.47
1438.43
1333.15
1096.97
867.94
701.45
688.28
644.02
549.95

FIG. 6

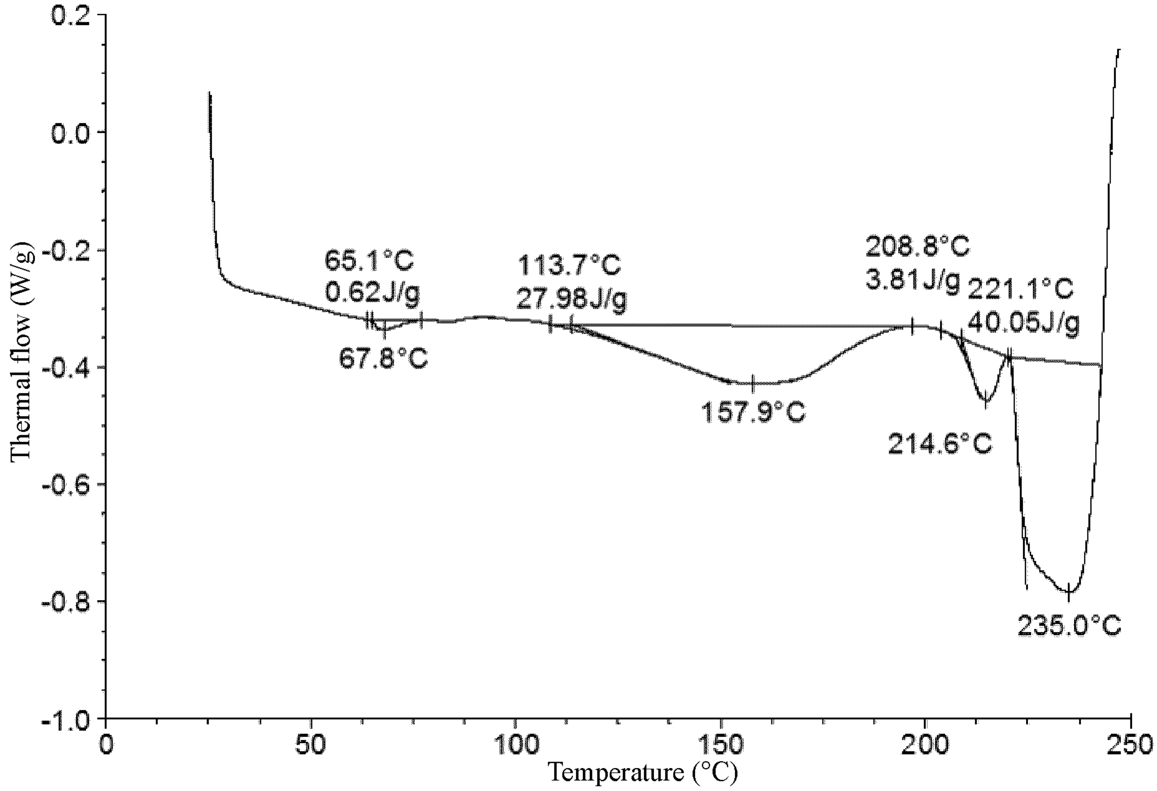
Thermal flow (W/g)
Temperature (°C)
65.1°C
0.62J/g
67.8°C
113.7°C
27.98J/g
157.9°C
208.8°C
3.81J/g
221.1°C
40.05J/g
214.6°C
235.0°C

FIG. 7

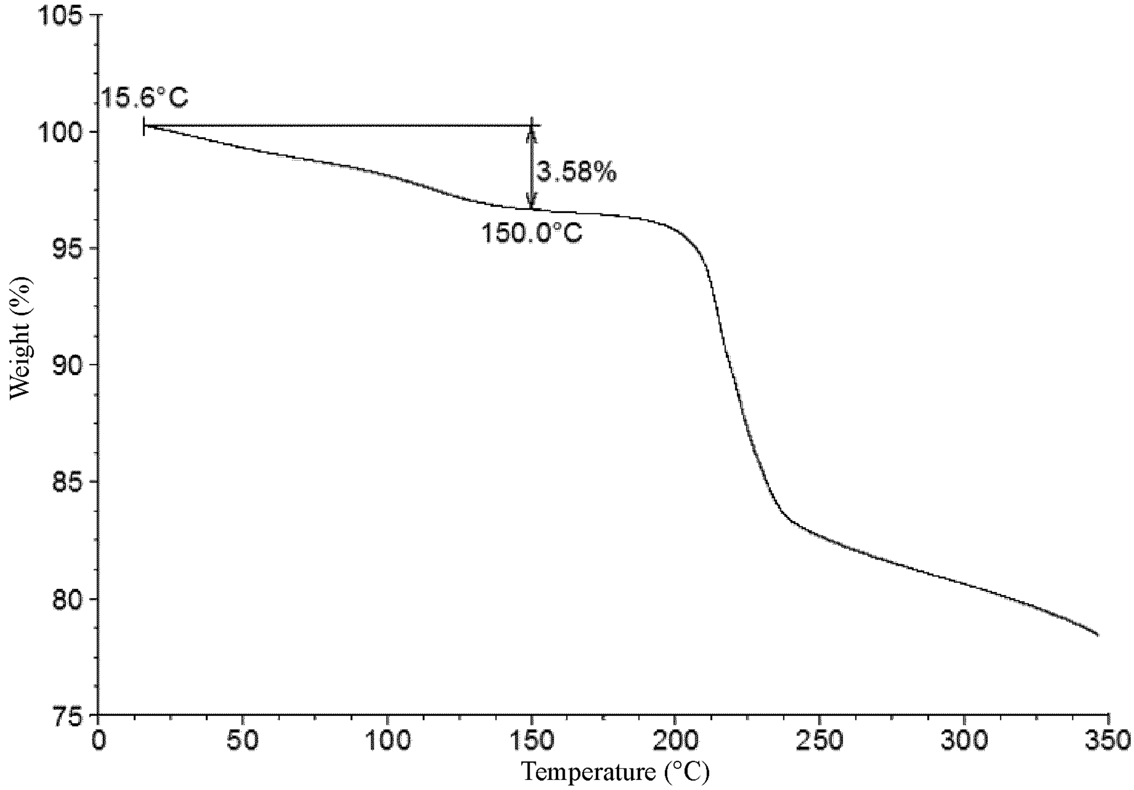
105
100
95
90
85
80
75
Weight (%)
15.6°C
3.58%
150.0°C
0
50
100
150
200
250
300
350
Temperature (°C)

FIG. 8

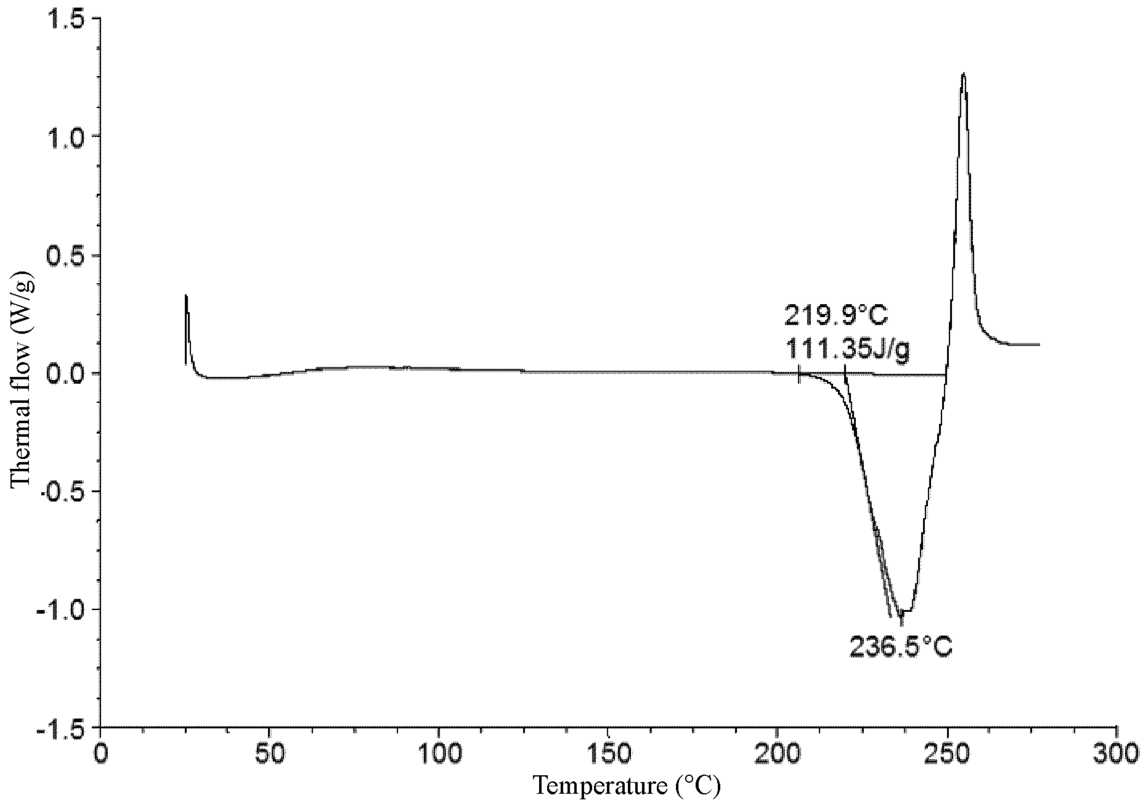
1.5
1.0
0.5
0.0
-0.5
-1.0
-1.5
Thermal flow (W/g)
219.9°C
111.35J/g
236.5°C
0
50
100
150
200
250
300
Temperature (°C)

FIG. 9

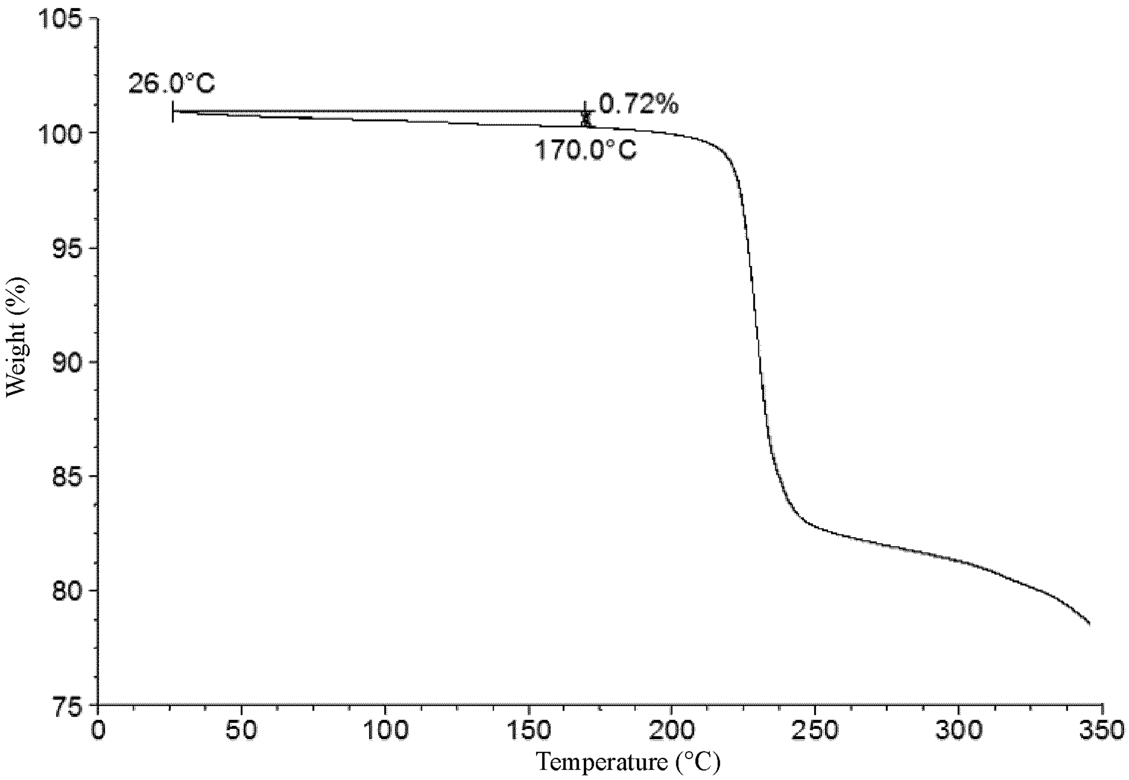


FIG. 10

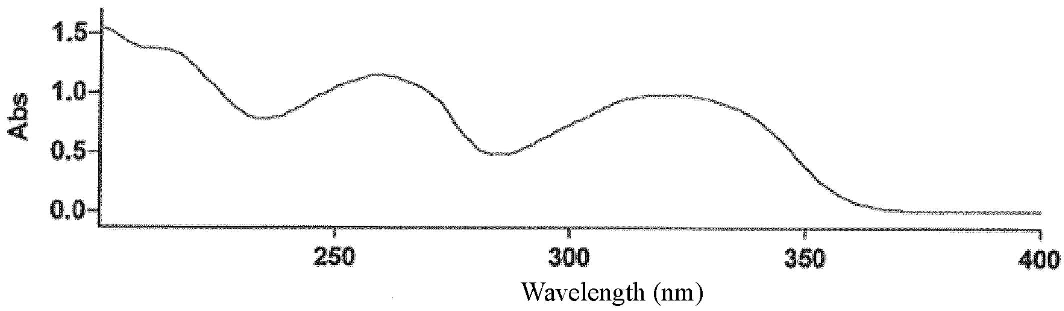


FIG. 11

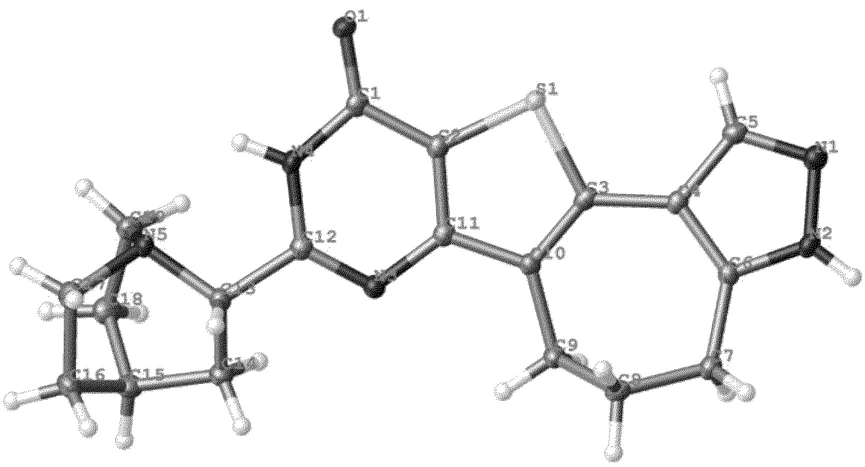

FIG. 12

**INTERNATIONAL SEARCH REPORT**

International application No.
**PCT/CN2021/134435**

**A. CLASSIFICATION OF SUBJECT MATTER**

C07D 519/00(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D519/-; A61K31/-; A61P35/-

Documentation searched other than minimum documentation to the extent that such documents are included in the fields searched

Electronic data base consulted during the international search (name of data base and, where practicable, search terms used)

WPI, EPODOC, CNPAT, CNKI, Caplus(STN), Registry(STN): CDC7, 抑制剂, 晶体, cell division cycle, inhibit+, crystal, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2020239107 A1 (CHIA TAI TIANQING PHARMACEUTICAL GROUP CO., LTD. et al.) 03 December 2020 (2020-12-03)<br>embodiment 1, claims 14-15 | 1-14 |
| A | CN 108779126 A (TAKEDA PHARMACEUTICAL COMPANY LIMITED) 09 November 2018 (2018-11-09)<br>description, paragraphs [0016]-[0020] | 1-14 |
| A | CN 102844320 A (TAKEDA PHARMACEUTICAL COMPANY LIMITED) 26 December 2012 (2012-12-26)<br>claims 1-20 | 1-14 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 February 2022** | **28 February 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/134435**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

[1] Claim 14 relates to a method for treating a disease. However, a search has been made on the basis of uses of the described compound in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/134435**

| Patent document cited in search report | Publication date (day/month/year) | Patent family member(s) | Publication date (day/month/year) |
|---|---|---|---|
| WO 2020239107 A1 | 03 December 2020 | AU 2020281411 A1 | 06 January 2022 |
| | | CA 3142202 A1 | 03 December 2020 |
| | | CN 113874379 | 31 December 2021 |
| CN 108779126 A | 09 November 2018 | CA 3016708 A1 | 05 October 2017 |
| | | PL 3436463 T3 | 08 November 2021 |
| | | JP 2020143076 A | 10 September 2020 |
| | | DK 3436463 T3 | 30 August 2021 |
| | | JP 2019510033 A | 11 April 2019 |
| | | JP 6918824 B2 | 11 August 2021 |
| | | PT 3436463 T | 06 September 2021 |
| | | WO 2017172565 A1 | 05 October 2017 |
| | | TW 201736383 A | 16 October 2017 |
| | | TW I738748 B | 11 September 2021 |
| | | ES 2884398 T3 | 10 December 2021 |
| | | EP 3436463 A1 | 06 February 2019 |
| | | EP 3436463 B1 | 23 June 2021 |
| CN 102844320 A | 26 December 2012 | CA 2790284 A1 | 25 August 2011 |
| | | CA 2790284 C | 08 January 2019 |
| | | MY 164776 A | 30 January 2018 |
| | | SI 2540728 T1 | 28 June 2019 |
| | | US 2013029969 A1 | 31 January 2013 |
| | | US 8722660 B2 | 13 May 2014 |
| | | CR 20120448 A | 13 November 2012 |
| | | SG 183304 A1 | 27 September 2012 |
| | | DK 2540728 T3 | 13 May 2019 |
| | | PL 2540728 T3 | 30 September 2019 |
| | | IL 221442 D0 | 31 October 2012 |
| | | US 2016310494 A1 | 27 October 2016 |
| | | US 9655900 B2 | 23 May 2017 |
| | | US 2015158882 A1 | 11 June 2015 |
| | | US 9388195 B2 | 12 July 2016 |
| | | PE 20130184 A1 | 09 March 2013 |
| | | MA 34064 B1 | 05 March 2013 |
| | | CO 6592104 A2 | 02 January 2013 |
| | | EP 2540728 A1 | 02 January 2013 |
| | | EP 2540728 A4 | 06 November 2013 |
| | | EP 2540728 B1 | 10 April 2019 |
| | | ES 2733221 T3 | 28 November 2019 |
| | | NZ 602089 A | 30 May 2014 |
| | | EA 201290800 A1 | 29 March 2013 |
| | | EA 020724 B1 | 30 January 2015 |
| | | RS 58796 B1 | 31 July 2019 |
| | | PT 2540728 T | 11 July 2019 |
| | | JP WO2011102399 A1 | 17 June 2013 |
| | | JP 5689454 B2 | 25 March 2015 |
| | | KR 20130001246 A | 03 January 2013 |
| | | KR 101735868 B1 | 15 May 2017 |
| | | CL 2012002250 A1 | 08 February 2013 |
| | | HU E043514 T2 | 28 August 2019 |
| | | US 2014228352 A1 | 14 August 2014 |

Form PCT/ISA/210 (patent family annex) (January 2015)

**INTERNATIONAL SEARCH REPORT**
**Information on patent family members**

International application No.
**PCT/CN2021/134435**

| Patent document cited in search report | Publication date (day/month/year) | Patent family member(s) | | | Publication date (day/month/year) |
|---|---|---|---|---|---|
| | | US | 8921354 | B2 | 30 December 2014 |
| | | DO | P2012000224 | A | 31 January 2013 |
| | | LT | 2540728 | T | 25 June 2019 |
| | | MX | 2012009602 | A | 17 December 2012 |
| | | MX | 353500 | B | 16 January 2018 |
| | | TN | 2012000401 | A1 | 30 January 2014 |
| | | EC | SP12012160 | A | 28 March 2013 |
| | | CN | 102844320 | B | 23 March 2016 |
| | | US | 2017209452 | A1 | 27 July 2017 |
| | | ME | 03410 | B | 20 January 2020 |
| | | EP | 3533797 | A1 | 04 September 2019 |
| | | HR | P20190947 | T1 | 26 July 2019 |
| | | AU | 2011216404 | A1 | 20 September 2012 |
| | | AU | 2011216404 | B2 | 07 April 2016 |
| | | US | 2014235615 | A1 | 21 August 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011383418